# EUROPEAN PATENT SPECIFICATION

(11) **EP 4 359 400 B1**
(45) Date of publication and mention of the grant of the patent: **02.07.2025**
(21) Application number: 22744048.4
(22) Date of filing: 22.06.2022
(51) Int. Cl.: C07D 401/14, C07C 51/43, C07C 55/10, C07C 55/12, C07C 57/15, A61P 35/00

(54) **SALT AND CRYSTAL FORM OF AN AZETIDINYL SUBSTITUTED ISOQUINLINE ACTING AS EPIDERMAL GROWTH FACTOR RECEPTOR INHIBITOR FOR THE TREATMENT OF CANCER**
SALZ- UND KRISTALLFORMEN EINES AZETIDINYL SUBSTITUIERTEN ISOCHINOLINS ALS EPIDERMALER WACHSTUMSFAKTORREZEPTORHEMMER ZUR BEHANDLUNG VON KREBS
FORMES DE SEL ET FORMES CRISTALLINES D'UN ISOQUINOLINE SUBSTITUÉ PAR AZETIDINE EN TANT QU'INHIBITEUR DU RÉCEPTEUR DU FACTEUR DE CROISSANCE ÉPIDERMIQUE POUR LE TRAITEMENT DES MALADIES DE CANCER

(30) Priority: 23.06.2021 US 202163214089 P
(43) Date of publication of application: 01.05.2024
(73) Proprietor: Blueprint Medicines Corporation, Cambridge, MA 02139 (US)
(72) Inventor: BUTLER, Erika, Halifx, NS B3J 3M5 (CA); KINKEMA, Caitlin, N., Cambridge, MA 02139 (US); LEE, Christopher, Cambridge, MA 02139 (US)
(74) Representative: Mathys & Squire
(86) International application number: PCT/US2022/034550
(87) International publication number: WO 2022/271846

(56) References cited:
- WO-A1-2017/120429
- WO-A1-2021/133809

## Description

### BACKGROUND

Epidermal Growth Factor Receptor (EGFR) is a member of the erbB receptor family, which includes transmembrane protein tyrosine kinase receptors. When binding to a ligand such as epidermal growth factor (EGF), EGFR is able to form a homodimer on the cell membrane or with other members of the erbB family. The formation of these dimers often causes tyrosine phosphorylation, and can lead to the activation or alteration of various downstream cellular pathways, including cell proliferation, survival, and anti-apoptosis. Because of this cellular function, disorders in EGFR signaling, including increased expression of EGFR or its ligands and deletions or mutations in the EGFR gene or protein, can activate cell growth or mitigate normal apoptosis mechanisms and pathway, traditional hallmarks of tumor cell growth and proliferation. For example, EFGR mutations or deletions are commonly found in non-small cell lung cancer (NSCLC) tumors.

The two most frequent EGFR alternations found in NSCLC tumors are 1) short in-frame deletions in exon 19 (del19) and 2) L858R, a single missense mutation in exon 21 (Cancer Discovery 2016 6(6) 601). These two alterations cause ligand-independent EGFR activation and are referred to as primary or activating mutations in EGFR mutant NSCLC (EGFR M+). Clinical experience shows an objective response rate (ORR) of approximately 60-85% in EGFR M+ NSCLC patients treated first line (1L) with EGFR tyrosine kinase inhibitors (TKIs) erlotinib, gefitinib, afatinib and osimertinib (Lancet Oncol. 2010 Vol. 11, 121*;* Lancet Oncol. 2016 Vol. 17, 577; N. Engl. J. Med. 2017 Nov 18 Doi:10.1056/NEJMoa1713137; Lancet Oncol. 2011 Vol. 12, 735). These findings show that EGFR mutant NSCLC tumors are dependent on the oncogenic activity of the mutated EGFR and that del19 and L858R are the oncogenic mutation in this disease, which validates these as drug targets and biomarkers for many forms of NSCLC.

However, after an average of 10-12 months of treatment with first generation (erlotinib and gefitinib) and second generation (afatinib) EGFR TKIs, resistance to these small molecule inhibitors has been observed in almost all NSCLC patients *(*Lancet Oncol. 2010 Feb;11(2):121-8.; Lancet Oncol. 2016 May;17(5):577-89; Lancet Oncol. 2011 Aug;12(8):735-42). The most common resistance mechanism is the development of a second EGFR mutation, T790M, which occurs in 50 % to 70 % of patients progressing on 1^{st} and 2^{nd} generation EGFR inhibitors. (Blakely et al., Cancer Discov; 2012, 2(10); 872-5; Kobayashi et al., Cancer Res 2005; 65: (16)). This secondary mutation reduces the affinity of the drug with the target, thereby producing drug resistance, and resulting in tumor recurrence or disease progression.

Due to the prevalence of the T790M mutation, a number of companies have attempted to develop new small molecule EGFR inhibitors for treating patients with the drug-resistant mutant. For example, osimertinib (Tagrisso^{®}), a third generation EGFR TKI, has been developed to treat NSCLC patients if the cancer cells are positive for the primary EGFR mutations del19 or L858R with or without the T790M mutation in the gene coding for EGFR. Although the third generation EGFR TKI, osimertinib, has shown efficacy on NSCLC patients, unfortunately, resistance mediated by an exon 20 C797 mutation (often C797S) in EGFR usually develops within approximately 10 months (European Journal of Medicinal Chemistry 2017 Vol. 142: 32-47) and accounts for the majority of osimertinib resistance cases (Cancer Letters 2016 Vol. 385: 51-54). The EGFR del19/L858R T790M C797S cis mutant kinase variant typically emerges in second line (2L) patients following treatment with osimertinib and is often referred to as "triple mutant" EGFR and it can no longer be inhibited by first, second, or third generation EGFR inhibitors.

No approved EGFR TKI can inhibit the triple mutant variant. Therefore, there is a need to develop new EGFR inhibitors, which can inhibit with high selectivity EGFR mutants with the triple mutant, del19/L858R T790M C797S, while at the same time have no or low activity to wild-type EGFR. In addition to treating a mutant form of EGFR for which there is no current therapy, such selective EGFR inhibitors are likely to be more suitable as therapeutic agents, particularly for the treatment of cancer, due to reduction of toxicologies (diarrhea, skin rash) associated with wild-type EGFR inhibition.

WO2021/133809 discloses inhibitors of "triple mutant" EGFR, which can be used to treat various cancer, such as NSCLC. The structure of one of the inhibitors disclosed in WO2021/133809 referred to herein as "Compound (I)" is shown below:

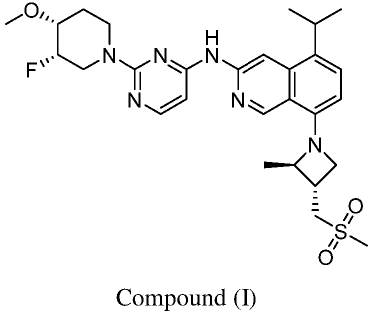

There is a need to develop new salt forms and/or solid forms of Compound (I) that are suitable to large scale manufacture and commercialization.

### SUMMARY

The present disclosure is directed to i) novel pharmaceutically acceptable salts of Compound (I) *(e.g.,* 1:0.5 Compound (I) Semi-Succinate, 1:0.5 Compound (I) Semi-Glutarate, 1:1 Compound (I) Fumarate) including the corresponding solid forms; and ii) novel solid forms of the free base of Compound (I) (hereinafter collectively referred to as "Salt or Solid Forms of the Disclosure").

The designation "1:0.5" is the molar ratio between Compound (I) and the acid (succinic acid or glutaric acid), and the designation "1:1" is the molar ratio between Compound (I) and the acid (fumaric acid).

In one aspect, the present disclosure provides a succinate salt of Compound (I), wherein the molar ratio between Compound (I) and succinic acid is 1:0.5. As noted above, this salt is also referred herein as "1:0.5 Compound (I) Semi-Succinate".

In another aspect, the present disclosure provides a glutarate salt of Compound (I), wherein the molar ratio between Compound (I) and glutaric acid is 1:0.5. As noted above, this salt is also referred herein as "1:0.5 Compound (I) Semi-Glutarate".

In another aspect, the present disclosure provides a fumarate salt of Compound (I), wherein the molar ratio between Compound (I) and fumaric acid is 1:1. As noted above, this salt is also referred herein as "1:1 Compound (I) Fumarate".

In another aspect, the present disclosure provides a first polymorph of the free base of Compound (I). This first polymorph is also referred herein as "Compound (I) Free Base Form A".

In another aspect, the present disclosure provides a second polymorph of the free base of Compound (I). This second polymorph is also referred herein as "Compound (I) Free Base Form B".

In another aspect, the present disclosure provides a pharmaceutical composition comprising 1:0.5 Compound (I) Semi-Succinate, 1:0.5 Compound (I) Semi-Glutarate, 1:1 Compound (I) Fumarate, Compound (I) Free Base Form A, or Compound (I) Free Base Form B, and a pharmaceutically acceptable carrier or diluent.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present disclosure provides a method of treating or ameliorating cancer in a subject, comprising administering to the subject in need thereof a pharmaceutically effective amount of the salt or free base form disclosed herein or the corresponding pharmaceutical composition. In some aspects, the cancer which is treated or ameliorated is non-small cell lung cancer.

The present disclosure also provides a method of inhibiting aberrant EGFR activity in a subject, comprising administering to the subject in need thereof a pharmaceutically effective amount of the salt or free base disclosed herein or the corresponding pharmaceutical composition.

The present disclosure provides a method of inhibiting various mutated forms of EGFR, including EGFR enzymes with amino acid modification selected from the group consisting of L858R, T790M, C797S, and combinations thereof.

### BRIEF DESCRIPTION OF THE DRAWINGS

Figure 1 shows the X-ray Powder Diffraction (XRPD) pattern of 1:0.5 Compound (I) Semi-Succinate Form C.
Figure 2 shows the Thermogravimetric Analysis (TGA) and Differential Scanning Calorimetry Analysis (DSC) thermograms of 1:0.5 Compound (I) Semi-Succinate Form C.
Figure 3 shows the Differential Scanning Calorimetry Analysis (DSC) thermogram of 1:0.5 Compound (I) Semi-Succinate Form C.
Figure 4 shows the X-ray Powder Diffraction (XRPD) pattern of 1:0.5 Compound (I) Semi-Glutarate Form D.
Figure 5 shows the Thermogravimetric Analysis (TGA) and Differential Scanning Calorimetry Analysis (DSC) thermograms of 1:0.5 Compound (I) Semi-Glutarate Form D.
Figure 6 shows the Differential Scanning Calorimetry Analysis (DSC) thermogram of 1:0.5 Compound (I) Semi- Glutarate Form D.
Figure 7 shows the X-ray Powder Diffraction (XRPD) pattern of 1:1 Compound (I) Fumarate Form E.
Figure 8 shows the Thermogravimetric Analysis (TGA) and Differential Scanning Calorimetry Analysis (DSC) thermograms of 1:1 Compound (I) Fumarate Form E.
Figure 9 shows the Differential Scanning Calorimetry Analysis (DSC) thermogram of 1:1 Compound (I) Fumarate Form E.
Figure 10 shows the X-ray Powder Diffraction (XRPD) pattern of Compound (I) Freebase Form A.
Figure 11 shows the Thermogravimetric Analysis (TGA) and Differential Scanning Calorimetry Analysis (DSC) thermograms of Compound (I) Freebase Form A.
Figure 12 shows the X-ray Powder Diffraction (XRPD) pattern of Compound (I) Freebase Form B.
Figure 13 shows the Thermogravimetric Analysis (TGA) and Differential Scanning Calorimetry Analysis (DSC) thermograms of Compound (I) Freebase Form B.
Figure 14 shows the Differential Scanning Calorimetry Analysis (DSC) thermogram of Compound (I) Freebase Form B.
Figure 15 shows the X-ray Powder Diffraction (XRPD) pattern of Amorphous Compound (I).
Figure 16 shows the Thermogravimetric Analysis (TGA) and Differential Scanning Calorimetry Analysis (DSC) thermograms of Amorphous Compound (I).
Figure 17 shows the Differential Scanning Calorimetry Analysis (DSC) thermogram of Amorphous Compound (I).

### DETAILED DESCRIPTION

The present disclosure is directed to the succinate salt (*i.e.,* 1:0.5 Semi-Succinate salt) of Compound (I), the glutarate salt (*i.e.,* 1:0.5 Semi-Glutarate) of Compound (I), the fumarate salt (*i.e.,* 1:1 Fumarate Salt) of Compound (I), freebase Form A of Compound (I), and freebase Form B of Compound (I).

As used herein, "crystalline" refers to a solid having a crystal structure wherein the individual molecules have a highly homogeneous regular three dimensional configuration.

In some embodiments, for the crystalline forms of Compound (I) salt or free base disclosed herein, at least a particular percentage by weight of the 1:0.5 or 1:1 Compound (I) salt or free base is in a particular crystalline form. Particular weight percentages include 85%, 86%, 87%, 88%, 89%, 90%, 91%, 92%, 93%, 94%, 95%, 96%, 97%, 98%, 99%, 99.5%, 99.9%, or a weight percentage of 70%-75%, 75%-80%, 80%-85%, 85%-90%, 90%-95%, 95%-100%, 70-80%, 80-90%, 90-100% by weight of the Compound (I) salt or free base is in a particular crystalline form. It is to be understood that all values and ranges between these values and ranges are meant to be encompassed by the present disclosure.

When the crystalline Compound (I) salt or free base is defined as a specified percentage of one particular crystal form of the Compound (I) salt or free base, the remainder is made up of amorphous form and/or crystal forms other than the one or more particular forms that are specified. Examples of particular crystalline forms include 1:0.5 Compound (I) Semi-Succinate Form C, 1:0.5 Compound (I) Semi-Glutarate Form D, 1:1 Compound (I) Fumarate Form E, Compound (I) Freebase Form A, and Compound (I) Freebase Form B, each of which are characterized by one or more properties as discussed herein.

Compound (I) has 4 chiral centers. Compound (I) in the salts and free base polymorphs disclosed herein is at least 80%, 90%, 99% or 99.9% by weight pure relative to the other stereoisomers, *i.e.,* the ratio of the weight of the stereoisomer over the weight of all the stereoisomers.

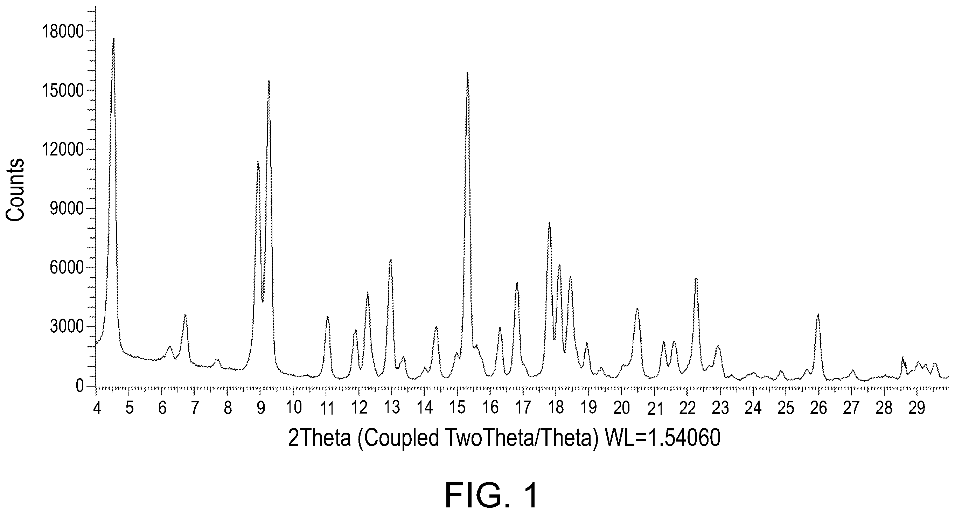

The crystalline Compound (I) salts disclosed herein exhibit strong, unique XRPD patterns with sharp peaks corresponding to angular peak positions in 2θ and a flat baseline, indicative of a highly crystalline material (*e.g.,* see Figure 1).

As used herein, an X-ray powder diffractogram is "substantially similar to that in [a particular] Figure" when at least 90%, such as at least 95%, at least 98%, or at least 99%, of the signals in the two diffractograms are the same± 0.2 °2θ. In determining "substantial similarity," one of ordinary skill in the art will understand that there may be variation in the intensities and/or signal positions in XRPD diffractograms even for the same crystalline form. Thus, those of ordinary skill in the art will understand that the signal maximum values in XRPD diffractograms (in degrees two-theta (°2θ) referred to herein) generally mean that value reported ±0.2 degrees 2θ of the reported value, an art-recognized variance discussed above.

### Succinate Salt of Compound (I)

In some embodiments, the present disclosure provides a succinate salt of Compound (I), represented by the following structural formula: 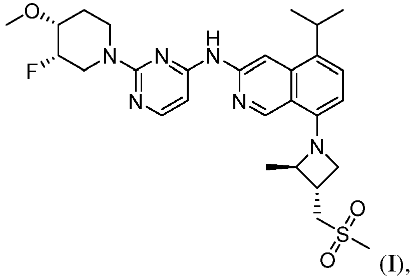 and wherein the molar ratio between compound (I) and succinic acid is 1:0.5.

In some embodiments, the succinate salt is crystalline.

In some embodiments, 1:0.5 Compound (I) Semi-Succinate is crystalline Form C, characterized by an X-ray powder diffraction pattern which comprises peaks at 4.5°, 9.3°, and 15.3° ± 0.2 in 2θ. In some embodiments, Form C is characterized by an X-ray powder diffraction pattern which comprises at least three peaks chosen from 4.5°, 8.9°, 9.3°, 15.3°, and 17.8° ± 0.2 in 2θ. In some embodiments, Form C is characterized by an X-ray powder diffraction pattern which comprises peaks at 4.5°, 8.9°, 9.3°, 15.3°, and 17.8° ± 0.2 in 2θ. In some embodiments, Form C is characterized by an X-ray powder diffraction pattern which comprises peaks at 4.5°, 8.9°, 9.3°, 13.0°, 15.3°, 16.8°, 17.8°, 18.1°, 18.5°, and 22.3° ± 0.2 in 2θ. In some embodiments, Form C is characterized by an X-ray powder diffraction pattern which comprises peaks at 4.5°, 6.7°, 8.9°, 9.3°, 11.1°, 12.3°, 13.0°, 14.4°, 15.3°, 16.3°, 16.8°, 17.8°, 18.1°, 18.5°, 20.5°, 22.3°, and 26.0° ± 0.2 in 2θ. In some embodiments, Form C is characterized by an X-ray powder diffraction pattern substantially similar to Figure 1.

In some embodiments, Form C is characterized by a differential scanning calorimeter with an onset temperature (*i.e.,* the melting temperature) of 175 ± 2°C. In some embodiments, Form C is characterized by a differential scanning calorimeter with an onset temperature of
176 ± 2°C. In some embodiments, Form C is characterized by a differential scanning calorimeter with a peak temperature of 182 ± 2°C. In some embodiments, Form C is characterized by a differential scanning calorimeter with a peak temperature of 179 ± 2°C.

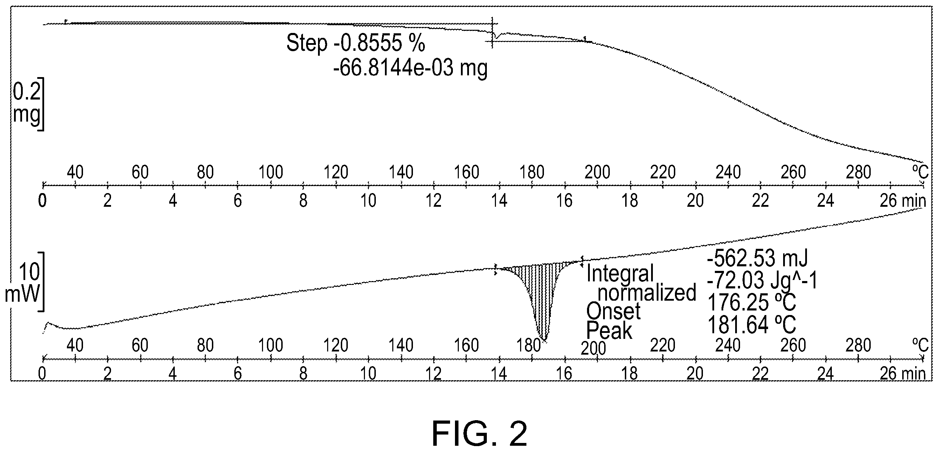

In some embodiments, Form C is characterized by a thermogravimetric analysis (TGA) substantially similar to Figure 2.

In some embodiments, at least 90%, 91%, 92%, 93%, 94%, 95%, 96%, 97%, 98%, 99%, 99.5%, or 99.9% by weight of 1:0.5 Compound (I) Semi-Succinate is in crystalline Form C.

### Glutarate Salt of Compound (I)

In some embodiments, the present disclosure provides a glutarate salt of Compound (I), represented by the following structural formula: 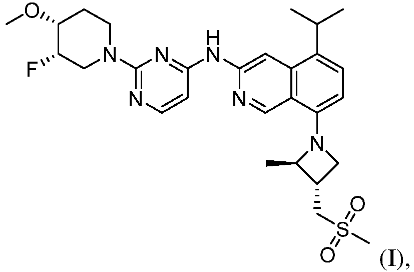 and wherein the molar ratio between compound (I) and glutaric acid is 1:0.5.

In some embodiments, the glutarate salt is crystalline.

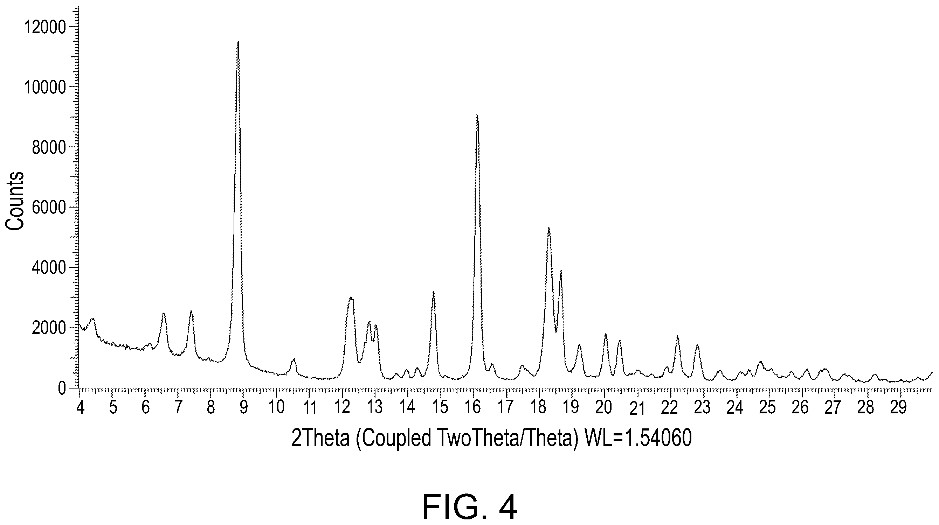

In some embodiments, 1:0.5 Compound (I) Semi-Glutarate crystalline Form D, characterized by an X-ray powder diffraction pattern which comprises peaks at 8.8°, 16.1°, and 18.3° ± 0.2 in 2θ. In some embodiments, Form D is characterized by an X-ray powder diffraction pattern which comprises at least three peaks chosen from 8.8°, 14.8°, 16.1°. 18.3°, and 18.7° ± 0.2 in 2θ. In some embodiments, Form D is characterized by an X-ray powder diffraction pattern which comprises peaks at 8.8°, 14.8°, 16.1°. 18.3°, and 18.7° ± 0.2 in 2θ. In some embodiments, Form D is characterized by an X-ray powder diffraction pattern which comprises peaks at 7.4°, 8.8°, 12.3°, 14.8°, 16.1°, 18.3°, and 18.7° ± 0.2 in 2θ. In some embodiments, Form D is characterized by an X-ray powder diffraction pattern which comprises peaks at 6.6°, 7.4°, 8.8°, 12.3°, 12.9°, 14.8°, 16.1°, 18.3°, 18.7°, 19.2°, 20.0°, and 22.2° ± 0.2 in 2θ. In some embodiments, Form D is characterized by an X-ray powder diffraction pattern substantially similar to Figure 4.

In some embodiments, Form D is characterized by a differential scanning calorimeter with an onset temperature of 142 ± 2°C. In some embodiments, Form D is characterized by a differential scanning calorimeter with a peak temperature of 150 ± 2°C. In some embodiments, Form D is characterized by a differential scanning calorimeter with a peak temperature of 148 ± 2°C.

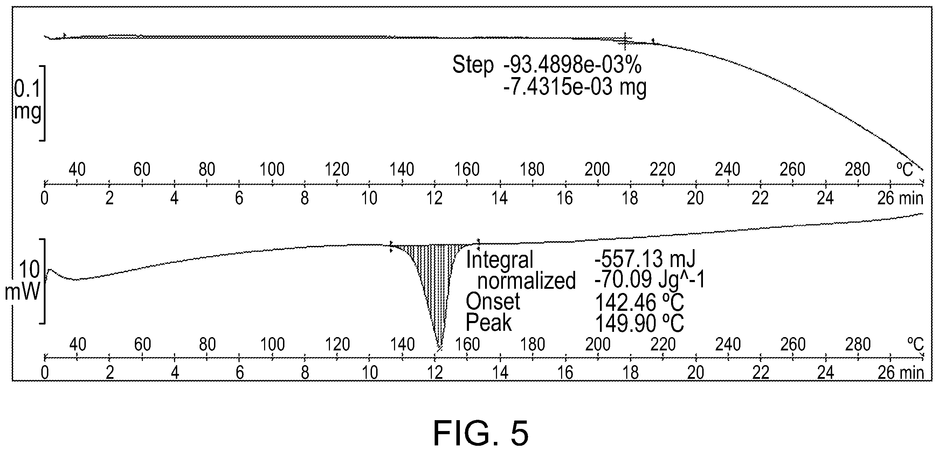

In some embodiments, Form D is characterized by a thermogravimetric analysis (TGA) substantially similar to Figure 5.

In some embodiments, at least 90%, 91%, 92%, 93%, 94%, 95%, 96%, 97%, 98%, 99%, 99.5%, or 99.9% by weight of 1:0.5 Compound (I) Semi-Glutarate is in crystalline Form D.

### Fumarate Salt of Compound (I)

In some embodiments, the present disclosure provides a fumarate salt of Compound (I), represented by the following structural formula: 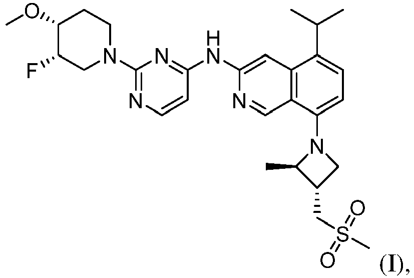 and wherein the molar ratio between compound (I) and fumaric acid is 1:1.

In some embodiments, the fumarate salt is crystalline.

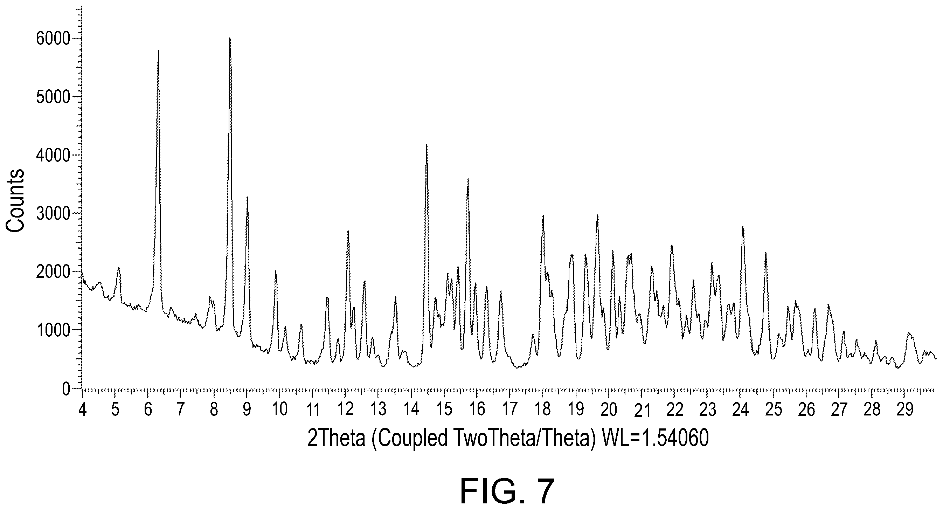

In some embodiments, 1:1 Compound (I) Fumarate is in crystalline Form E, characterized by an X-ray powder diffraction pattern which comprises peaks at 6.3°, 8.5°, and 14.5° ± 0.2 in 2θ. In some embodiments, Form E is characterized by an X-ray powder diffraction pattern which comprises at least three peaks chosen from 6.3°, 8.5°, 9.0°, 14.5°, 15.7°, and 18.0° ± 0.2 in 2θ. In some embodiments, Form E is characterized by an X-ray powder diffraction pattern which comprises peaks at 6.3°, 8.5°, 9.0°, 14.5°, 15.7°, and 18.0° ± 0.2 in 2θ. In some embodiments, Form E is characterized by an X-ray powder diffraction pattern which comprises peaks at 6.3°, 8.5°, 9.0°, 12.1°, 14.5°, 15.7°, 18.0°, 19.7°, 20.1°, and 21.9° ± 0.2 in 2θ. In some embodiments, Form E is characterized by an X-ray powder diffraction pattern which comprises peaks at 6.3°, 8.5°, 9.0°, 12.1°, 14.5°, 15.1°, 15.2°, 15.4°, 15.7°, 18.0°, 18.2°, 18.9°, 19.3°, 19.7°, 20.1°, 20.6°, 20.7°, 21.3°, and 21.9° ± 0.2 in 2θ. In some embodiments, Form E is characterized by an X-ray powder diffraction pattern substantially similar to Figure 7.

In some embodiments, Form E is characterized by a differential scanning calorimeter with an onset temperature of 164 ± 3°C. In some embodiments, Form E is characterized by a differential scanning calorimeter with an onset temperature of 165 ± 2°C. In some embodiments, Form E is characterized by a differential scanning calorimeter with an onset temperature of 162 ± 2°C. In some embodiments, Form E is characterized by a differential scanning calorimeter with a peak temperature of 171 ± 2°C.

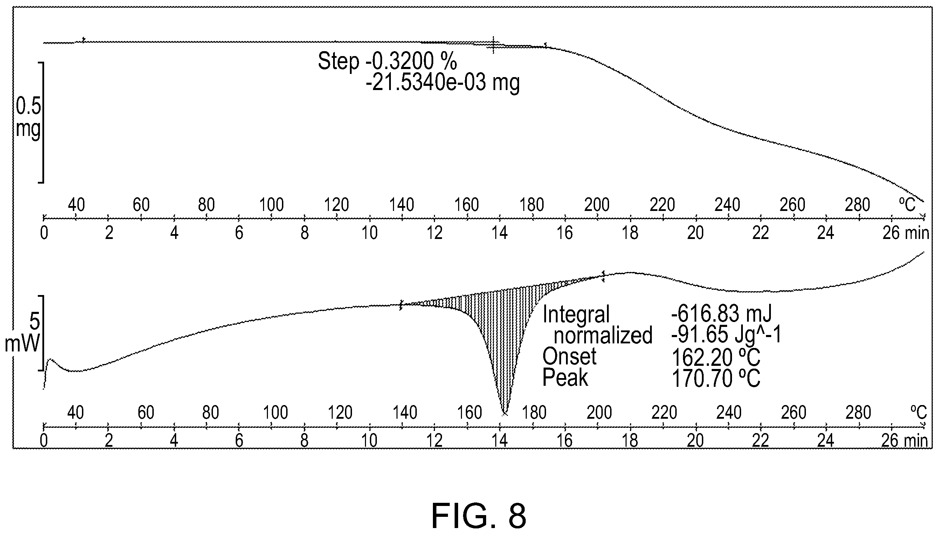

In some embodiments, Form E is characterized by a thermogravimetric analysis (TGA) substantially similar to Figure 8.

In some embodiments, at least 90%, 91%, 92%, 93%, 94%, 95%, 96%, 97%, 98%, 99%, 99.5%, or 99.9% by weight of 1:1 Compound (I) Fumarate is in crystalline Form E.

### Compound (I) Freebase Form A

In some embodiments, the present disclosure provides the freebase of Compound (I), represented by the following structural formula: 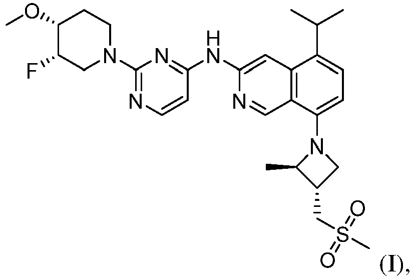 and wherein the freebase of Compound (I) is in a crystalline Form A.

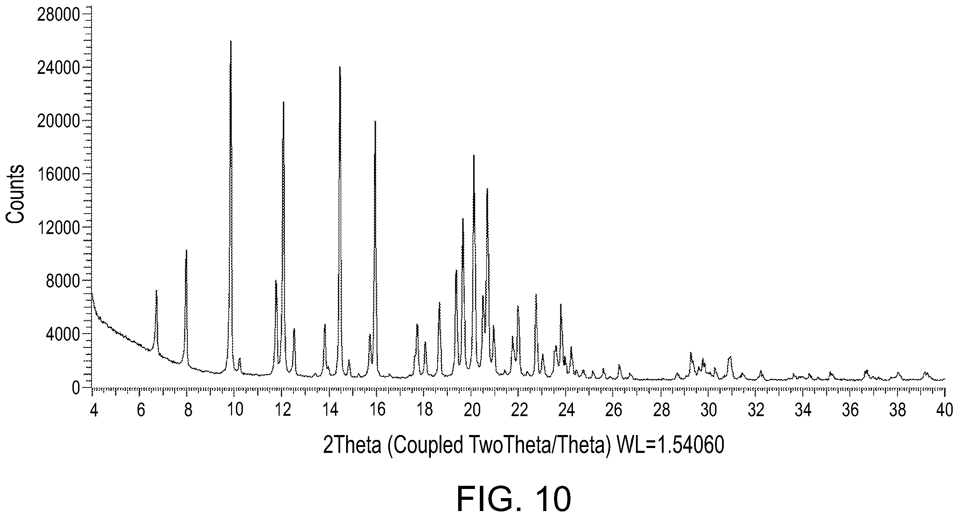

In some embodiments, Compound (I) Freebase is in a crystalline Form A, characterized by an X-ray powder diffraction pattern which comprises peaks at 9.9°, 12.1°, and 14.5° ± 0.2 in 2θ. In some embodiments, Form A is characterized by an X-ray powder diffraction pattern which comprises at least three peaks chosen from 9.9°, 12.1°, 14.5°, 15.9°, and 20.1° ± 0.2 in 2θ. In some embodiments, Form A is characterized by an X-ray powder diffraction pattern which comprises peaks at 9.9°, 12.1°, 14.5°, 15.9°, and 20.1° ± 0.2 in 2θ. In some embodiments, Form A is characterized by an X-ray powder diffraction pattern which comprises peaks at 8.0°, 9.9°, 11.8°, 12.1°, 14.5°, 15.9°, 19.4°, 19.7°, 20.1°, and 20.7° ± 0.2 in 2θ. In some embodiments, Form A is characterized by an X-ray powder diffraction pattern which comprises peaks at 6.7°, 8.0°, 9.9°, 11.8°, 12.1°, 14.5°, 15.9°, 18.7°, 19.4°, 19.7°, 20.1°, 20.5°, 20.7°, 22.0°, 22.8°, and 23.8° ± 0.2 in 2θ. In some embodiments, Form A is characterized by an X-ray powder diffraction pattern substantially similar to Figure 10.

In some embodiments, Form A is characterized by a differential scanning calorimeter with an onset temperature of 198 ± 2°C. In some embodiments, Form A is characterized by a differential scanning calorimeter with an onset temperature of 197 ± 2°C. In some embodiments, Form A is characterized by a differential scanning calorimeter with a peak temperature of 202 ± 2°C. In some embodiments, Form A is characterized by a differential scanning calorimeter with an onset temperature of 199 ± 2°C. In some embodiments, Form A is characterized by a differential scanning calorimeter with a peak temperature of 203 ± 2°C. In some embodiments, Form A is characterized by a differential scanning calorimeter with an onset temperature of 181 ± 2°C. In some embodiments, Form A is characterized by a differential scanning calorimeter with a peak temperature of 188 ± 2°C.

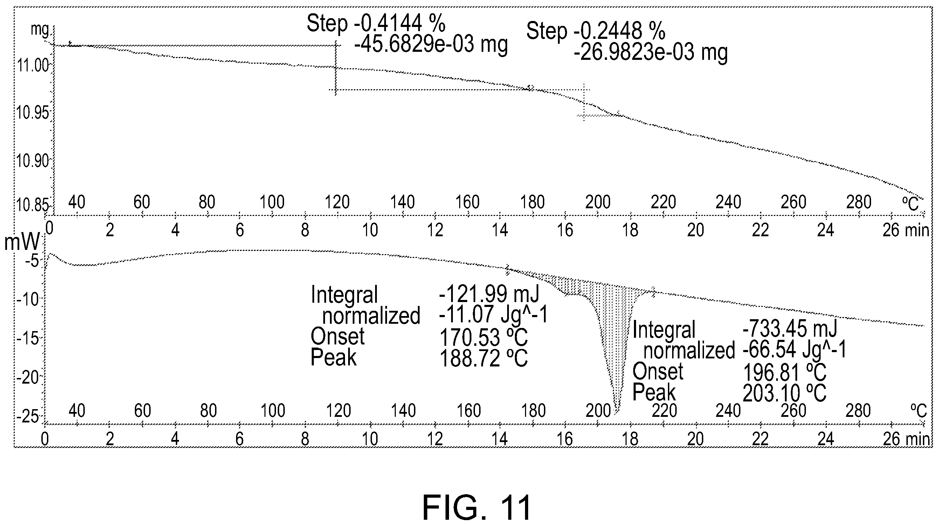

In some embodiments, Form A is characterized by a thermogravimetric analysis (TGA) substantially similar to Figure 11.

In some embodiments, at least 90%, 91%, 92%, 93%, 94%, 95%, 96%, 97%, 98%, 99%, 99.5%, or 99.9% by weight of Compound (I) Freebase is in crystalline Form A.

### Characterization of Compound (I) Freebase Form B

In some embodiments, the present disclosure provides the freebase of Compound (I), represented by the following structural formula: 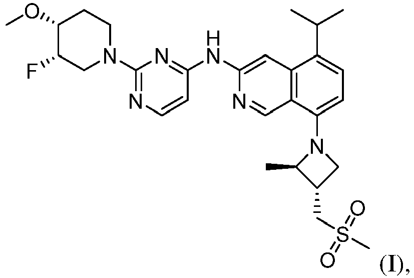 and wherein the freebase of Compound (I) is in a crystalline Form B.

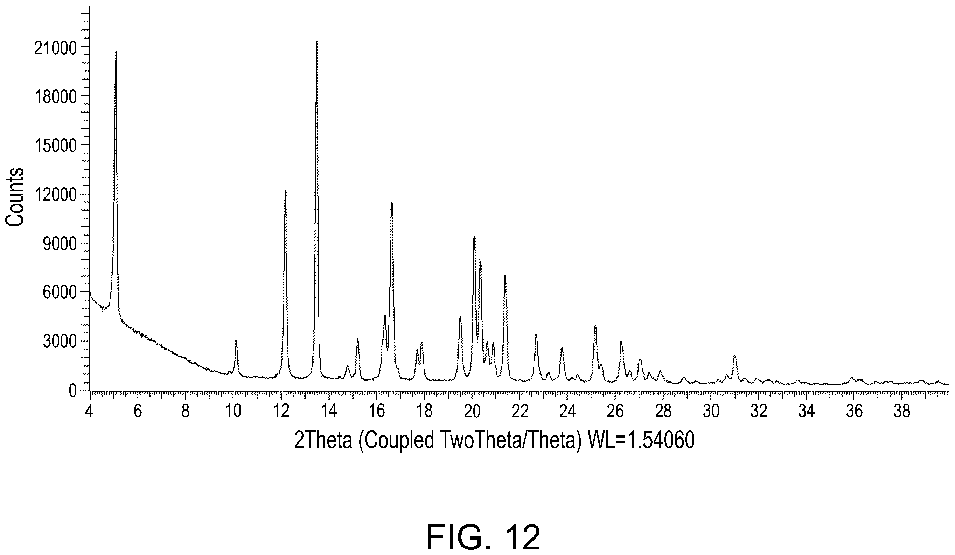

In some embodiments, Compound (I) Freebase is in crystalline Form B, characterized by an X-ray powder diffraction pattern which comprises peaks at 5.1°, 12.2°. 13.5°, 16.6°, and 20.1° ± 0.2 in 2θ. In some embodiments, Form B is characterized by an X-ray powder diffraction pattern which comprises peaks at 5.1°, 12.2°, 13.5°, 16.3°, 16.6°, 19.5°, 20.1°, 20.4°, 21.4°, 22.7°, and 25.2° ± 0.2 in 2θ. In some embodiments, Form B is characterized by an X-ray powder diffraction pattern which comprises peaks at 5.1°, 12.2°, 13.5°, 15.2°, 16.3°, 16.6°, 17.9°, 19.5°, 20.1°, 20.4°, 20.7°, 20.9°, 21.4°, 22.7°, 25.2°, and 26.3° ± 0.2 in 2θ. In some embodiments, Form B is characterized by an X-ray powder diffraction pattern substantially similar to Figure 12.

In some embodiments, Form B is characterized by a differential scanning calorimeter with an onset temperature of 158 ± 2°C. In some embodiments, Form B is characterized by a differential scanning calorimeter with an onset temperature of 159 ± 2°C. In some embodiments, Form B is characterized by a differential scanning calorimeter with a peak temperature of 165 ± 2°C. In some embodiments, Form B is characterized by a differential scanning calorimeter with a peak temperature of 166 ± 2°C.

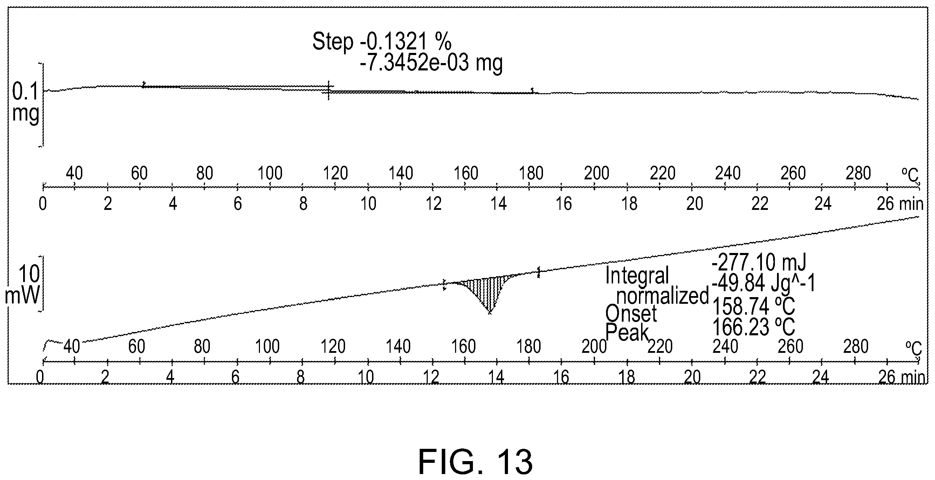

In some embodiments, Form B is characterized by a thermogravimetric analysis (TGA) substantially similar to Figure 13.

In some embodiments, at least 90%, 91%, 92%, 93%, 94%, 95%, 96%, 97%, 98%, 99%, 99.5%, or 99.9% by weight of Compound (I) Freebase is in crystalline Form B.

### Characterization of Amorphous Compound (I)

In some embodiments, the present disclosure provides amorphous form of Compound (I), represented by the following structural formula:

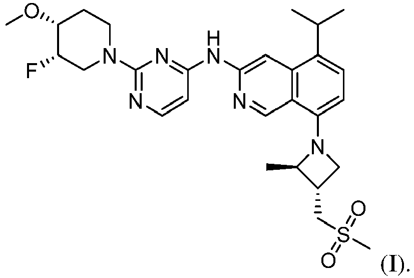

In some embodiments, the amorphous form is characterized by a differential scanning calorimeter with an onset temperature of 106 ± 2°C. In some embodiments, the amorphous form is characterized by a differential scanning calorimeter with an onset temperature of 109 ± 2°C. In some embodiments, the amorphous form is characterized by a differential scanning calorimeter with a peak temperature of 113 ± 2°C. In some embodiments, the amorphous form is characterized by a differential scanning calorimeter with a peak temperature of 114 ± 2°C.

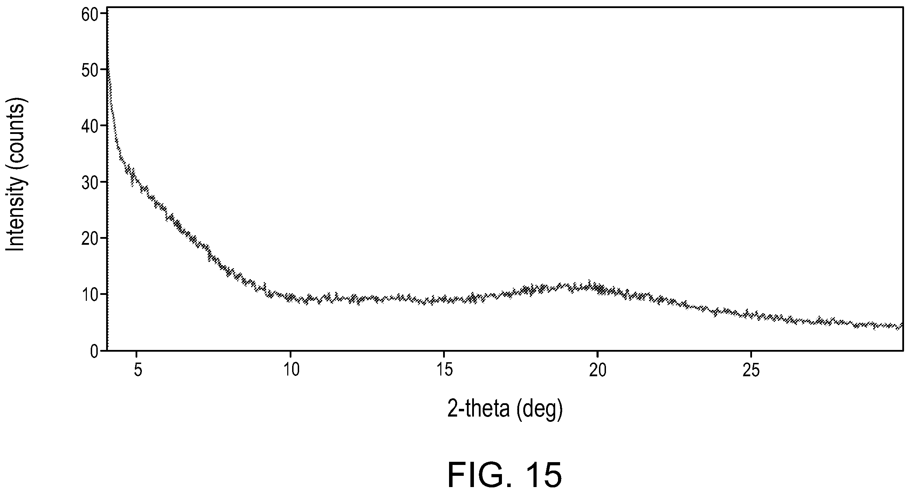

In some embodiments, the amorphous form is characterized by an X-ray powder diffraction pattern substantially similar to Figure 15.

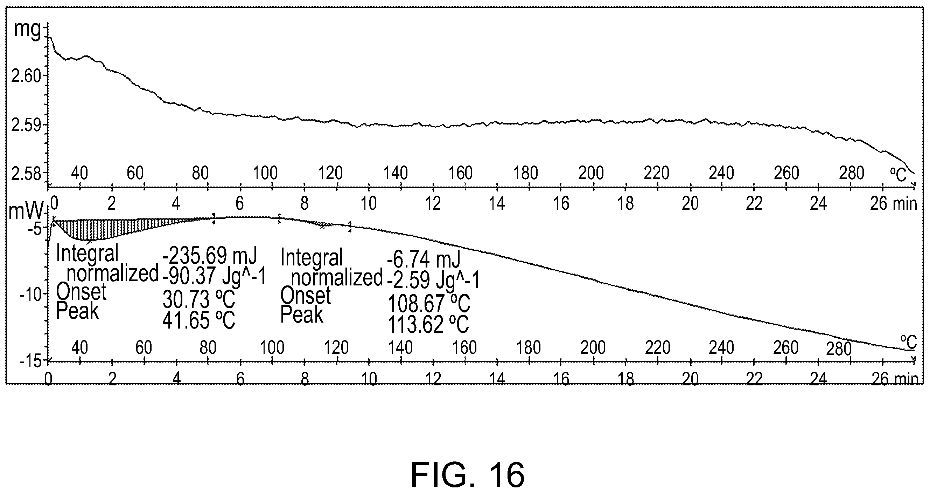

In some embodiments, the amorphous form is characterized by a thermogravimetric analysis (TGA) substantially similar to Figure 16.

In some embodiments, at least 90%, 91%, 92%, 93%, 94%, 95%, 96%, 97%, 98%, 99%, 99.5%, or 99.9% by weight of Compound (I) Freebase is in the amorphous form.

### Pharmaceutical Compositions

Pharmaceutical compositions of the disclosure (also referred to herein as the "disclosed pharmaceutical compositions") comprise a pharmaceutically acceptable carrier or diluent and a Salt or Solid Form of the Disclosure.

Some embodiments of the disclosure relate to a pharmaceutical composition comprising: a pharmaceutically acceptable excipient or a diluent; and a succinate salt of Compound (I), wherein the molar ratio between Compound (I) and succinic acid is 1:0.5. In some embodiments, the succinate salt is crystalline. In some embodiments, the succinate salt of Compound (I) is crystalline Form C.

Some embodiments of the disclosure relate to a pharmaceutical composition comprising: a pharmaceutically acceptable excipient or a diluent; and a glutarate salt of Compound (I), wherein the molar ratio between Compound (I) and glutaric acid is 1:0.5. In some embodiments, the glutarate salt is crystalline. In some embodiments, the glutarate salt of Compound (I) is crystalline Form D.

Some embodiments of the disclosure relate to a pharmaceutical composition comprising: a pharmaceutically acceptable excipient or a diluent; and a fumarate salt of Compound (I), wherein the molar ratio between Compound (I) and fumaric acid is 1:1. In some embodiments, the fumarate salt is crystalline. In some embodiments, the fumarate salt of Compound (I) is crystalline Form E.

Some embodiments of the disclosure relate to a pharmaceutical composition comprising: a pharmaceutically acceptable excipient or a diluent; and Compound (I) free base. In some embodiments, the free base is crystalline. In some embodiments, the free base of Compound (I) is crystalline Form A. In some embodiments, the free base of Compound (I) is crystalline Form B.

"A pharmaceutically acceptable carrier" or "a pharmaceutically acceptable diluent" refer to a substance that aids the formulation and/or administration of an active agent to and/or absorption by a subject and can be included in the pharmaceutical compositions of the disclosure without causing a significant adverse toxicological effect on the subject. Nonlimiting examples of pharmaceutically acceptable carriers and/or diluents include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, hydroxymethycellulose, fatty acid esters, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with or interfere with the activity of the compounds provided herein. One of ordinary skill in the art will recognize that other pharmaceutical excipients are suitable for use with Salt or Solid Form of the Disclosures or pharmaceutically acceptable salts thereof.

The pharmaceutical compositions of the disclosure optionally include one or more pharmaceutically acceptable carriers and/or diluents therefor, such as lactose, starch, cellulose and dextrose. Other excipients, such as flavoring agents, sweeteners, and preservatives, such as methyl, ethyl, propyl and butyl parabens, can also be included. More complete listings of suitable excipients can be found in the Handbook of Pharmaceutical Excipients (5th Ed., Pharmaceutical Press (2005)). A person skilled in the art would know how to prepare formulations suitable for various types of administration routes. Conventional procedures and ingredients for the selection and preparation of suitable formulations are described, for example, in Remington's Pharmaceutical Sciences (2003 - 20th edition) and in The United States Pharmacopeia: The National Formulary (USP 24 NF19) published in 1999. The carriers, diluents and/or excipients are "acceptable" in the sense of being compatible with the other ingredients of the pharmaceutical composition and not deleterious to the recipient thereof.

### Methods of Treatment

The present disclosure provides a method of inhibiting certain mutant forms of epidermal growth factor receptor (EGFR) in a subject in need thereof, comprising administering to the subject an effective amount of a Salt or Solid Form of the Disclosure or a pharmaceutical composition disclosed herein. Mutant forms of EGFR include for example, EGFR with LRTMCS mutation (the exon 19 deletion (del19) or exon 21 (L858R) substitution mutation, T790M mutation, and C797S mutation). Subjects "in need of inhibiting EGFR" are those having a disease for which a beneficial therapeutic effect can be achieved by inhibiting at least one mutant EGFR, *e.g.,* a slowing in disease progression, alleviation of one or more symptoms associated with the disease or increasing the longevity of the subject in view of the disease.

In some embodiments, the disclosure provides a method of treating a disease/condition/or cancer associated with or modulated by mutant EGFR, wherein the inhibition of the mutant EGFR is of therapeutic benefit, including but not limited to the treatment of cancer in a subject in need thereof. The method comprises administering to the subject an effective amount of a Salt or Solid Form of the Disclosure or pharmaceutical composition disclosed herein.

In another embodiment, the disclosure provides a method of treating a subject with cancer, comprising administering to the subject an effective amount of a Salt or Solid Form of the Disclosure or a pharmaceutical composition disclosed herein. Cancers to be treated according to the disclosed methods include lung cancer, colon cancer, urothelial cancer, breast cancer, prostate cancer, brain cancers, ovarian cancer, gastric cancer, pancreatic cancer, head and neck cancer, bladder cancer, and mesothelioma, including metastasis (in particular brain metastasis) of all cancers listed. Typically, the cancer is characterized by at one or more EGFR mutations described herein. In a specific embodiment, the cancer has progressed on or after EGFR tyrosine kinase inhibitor (TKI) Therapy. In a specific embodiment, the disease has progressed on or after first line osimertinib.

In a specific embodiment, the cancer to be treated is lung cancer. In a more specific embodiment, the cancer is non-small cell lung cancer (NSCLC). In some embodiments, the lung cancer is locally advanced or metastatic NSCLC, NSCLC adenocarcinoma, NSCLC with squamous histology and NSCLC with non-squamous histology. In another embodiment, the lung cancer is NSCLC adenocarcinoma. In another specific embodiment, the lung cancer (or non-small cell lung cancer) has metastasized to the brain.

In another embodiment, the disease/condition/or cancer associated with or modulated by mutant EGFR that is characterized by an EGFR genotype selected from genotypes 1-17 according the Table below (del18 = Exon 18 deletion, specifically, *e.g.,* del E709_T710 insD; del19 = Exon 19 deletion, specifically, *e.g.*, delE746_A750 (most common), delE746_S752insV, del747_A750insP, delL747_P753insS, and delS752_I759; ex20ins - Exon 20 insertion, specifically, *e.g*., D761-E762insX, A763-Y764insX, Y764-V765insX, V765-M766insX, A767-S768insX, S768-D769insX, V769-D770insX, N771-P772insX, P772-H773insX, H773-V774insX, and V774-C775insX):

**EGFR Genotype**

| | |
|---|---|
| 1 | EGFR del19 |
| 2 | EGFR del19 T790M |
| 3 | EGFR del19 C797S |
| 4 | EGFR del19 C797X (C797G or C797N) |
| 5 | EGFR del19 T790M C797S |
| 6 | EGFR del19 T790M C797S Q791P |
| 7 | EGFR del19 T790M (C797G or C797N) |
| 8 | EGFR del19 L792X (L792F, L792H or L792Y) |
| 9 | EGFR del19 T790M L792X (L792F, L792H, or L792Y) |
| 10 | EGFR del19 G796R (G796S) |
| 11 | EGFR del19 T790M G796R (G796S) C797S L792X (L792F, L792H or L792Y) |
| 12 | EGFR del19 L792R (L792V or L792P) |
| 13 | EGFR del19 L718Q (L718V) |
| 14 | EGFR del19 T790M L718Q (L718V) L792X (L792F, L792H or L792Y) |
| 15 | EGFR del19 T790M G796R (G796S) |
| 16 | EGFR del19 T790M L792R (L792V or L792P) |
| 17 | EGFR del19 T790M L718Q (L718V) |
| 18 | EGFR del19 T790M C797S L718Q (L718V) |
| 19 | EGFR del19 G724S |
| 20 | EGFR del19 T790M G724S |
| 21 | EGFR del19 S768I (SV768IL) |
| 22 | EGFR del19 T790M S768I (SV768IL) |
| 23 | EGFR del19 T790M C797S/G L792X (L792F, L792H, L792R, or L792Y) |
| 24 | EGFR del 19 V834L |
| 25 | EGFR del 19 T790M V834L |
| 27 | EGFR del19 T790M L792X (L792F, L792H, L792R, or L792Y) |
| 28 | EGFR del19 C797S L718Q (L718V) |
| 29 | EGFR del19 L718Q (L718V) A750P |
| 30 | EGFR del19 T790M L718Q (L718V) A750P L792V G796R |
| 31 | EGFR L858R |
| 32 | EGFR L858R T790M |
| 33 | EGFR L858R C797S |
| 34 | EGFR L858R C797X (797G or C797N) |
| 35 | EGFR L858R T790M C797S |
| 36 | EGFR L858R T790M C797S Q791P |
| 37 | EGFR L858R T790M C797X (C797G or C797N) |
| 38 | EGFR L858R L792X (L792F, L792H or L792Y) |
| 39 | EGFR L858R T790M L792X (L792F, L792H or L792Y) |
| 40 | EGFR L858R G796R (G796S) |
| 41 | EGFR L858R T790M G796R (G796S) C797S L792X (L792F, L792H or L792Y) |
| 42 | EGFR L858R L792R (L792V or L792P) |
| 43 | EGFR L858R L718Q (L718V) |
| 44 | EGFR L858R T790M G796R (G796S) |
| 45 | EGFR L858R T790M L792R (L792V or L792P) |
| 46 | EGFR L858R T790M L718Q (L718V) |
| 47 | EGFR L858R T790M C797S L718Q (L718V) |
| 48 | EGFR L858R T790M L718Q (L718V) L792X (L792F, L792H or L792Y) |
| 49 | EGFR L858R G724S |
| 50 | EGFR L858R T790M G724S |
| 51 | EGFR L858R S768I (SV768IL) |
| 52 | EGFR L858R T790M S768I (SV768IL) |
| 53 | EGFR L858R T790M C797S/G L792X (L792F, L792H, L792R, or L792Y) |
| 54 | EGFR L858R V834L |
| 55 | EGFR L858R T790M V834L |
| 57 | EGFR L858R T790M L792X (L792F, L792H, L792R, or L792Y) |
| 58 | EGFR L858R C797S L718Q (L718V) |
| 59 | EGFR L858R L718Q (L718V) A750P |
| 60 | EGFR L858R T790M L718Q (L718V) A750P L792V G796R |
| 61 | EGFR L861Q |
| 62 | EGFR L861Q T790M |
| 63 | EGFR L861Q T790M C797S/G/N |
| 64 | EGFR L861Q C797S/G/N |
| 65 | EGFR del18 |
| 66 | EGFR G719X (G719A, G719S, G719C, G719R, G719D, or G719V) |
| 67 | EGFR E709X (E709K, E709H, or E709A) |
| 68 | EGFR E709X (E709K, E709H, or E709A) (G719A, G719S, G719C, G719D, G719R, or G719V) |
| 69 | EGFR G719X (G719A, G719S, G719C, G719D, G719R, or G719V) S768I |
| 70 | EGFR ex20ins |
| 71 | EGFR ex20ins L718Q |
| 72 | EGFR ex20ins T790M |
| 73 | EGFR ex20ins C797S |
| 74 | EGFR S7681I |
| 75 | EGFR T790M |
| 76 | EGFR T790M C797S/G L792X (L792F, L792H, L792R, or L792Y) |

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 C797S.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 C797X (C797G or C797N).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M C797S.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M (C797G or C797N).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 L792X (L792F, L792H or L792Y).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M L792X (L792F, L792H, or L792Y).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 G796R (G796S).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 L792R (L792V or L792P).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 L718Q (L718V).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M G796R (G796S).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M L792R (L792V or L792P).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del19 T790M L718Q (L718V).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R T790M.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R C797S.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R C797X (797G or C797N).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R T790M C797S.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R T790M C797X (797G or C797N).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R L792X (L792F, L792H or L792Y).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R L790M L792X (L792F, L792H or L792Y).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R G796R (G796S).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R L792R (L792V or L792P).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R L718Q (L718V).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R T790M G796R (G796S).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R T790M L792R (L792V or L792P).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR L858R T790M L718Q (L718V).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR del18.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR G719X (G719A, G719S, G719C, G719R, G719D, or G719V).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR E709X (E709K, E709H, or E709A).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR E709X (E709K, E709H, or E709A) (G719A, G719S, G719C, G719D, G719R, or G719V).

In another embodiment, the disease/condition/or cancer (*e.g.,* NSCLC) being treated with a Salt or Solid Form of the Disclosure, or a pharmaceutical composition disclosed herein, is characterized by EGFR comprising EGFR G719X (G719A, G719S, G719C, G719D, G719R, or G719V) S768I.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR ex20ins.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR ex20ins L718Q.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR ex20ins T790M.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR ex20ins C797S.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR S7681I.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR T790M.

In another embodiment, the disease/condition/or cancer (*e.g.,* NSCLC) being treated with a Salt or Solid Form of the Disclosure, or a pharmaceutical composition disclosed herein, is characterized by EGFR comprising EGFR T790M C797S/G L792X (L792F, L792H, L792R, or L792Y).

In another embodiment, the disease/condition/or cancer (*e.g.,* NSCLC) being treated with a Salt or Solid Form of the Disclosure, or a pharmaceutical composition disclosed herein, is characterized by an EGFR genotype selected from genotypes 1-17.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to osimertinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to afatinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to dacomitinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to gefitinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to erlotinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to osimertinib and afatinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to osimertinib and dacomitinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to osimertinib and gefitinib.

In another embodiment, the disease/condition/or cancer (*e.g.,* NSCLC) being treated with a Salt or Solid Form of the Disclosure, or a pharmaceutical composition disclosed herein, is characterized by EGFR mutations that confer resistance to osimertinib and erlotinib.

Another embodiment is the treatment a subject with metastatic NSCLC with tumors harboring activating Exon 19 Deletion or L858R EGFR mutations as well as a resistance mutation disclosed herein as detected by an approved molecular testing methodology. Another embodiment is a Salt or Solid Form of the Disclosure, or a pharmaceutical composition disclosed herein, used in combination with a 1^{st} or 3^{rd} generation TKI indicated for the treatment of subject with metastatic NSCLC with tumors harboring T790M and C797S mutations as detected by an approved test, and whose disease has progressed on or after at least 2 prior EGFR TKI therapies.

Another embodiment is a Salt or Solid Form of the Disclosure, or a pharmaceutical composition disclosed herein, for the treatment of subjects with metastatic NSCLC whose disease with on-target EGFR resistance has progressed on or after any EGFR TKI. In a specific embodiment, the Salt or Solid Form of the Disclosure, or a pharmaceutical composition disclosed herein, is used in combination with a 1^{st} or 3^{rd} generation TKI indicated for the treatment of subject with metastatic NSCLC.

Another embodiment is a Salt or Solid Form of the Disclosure, or a pharmaceutical composition disclosed herein, for the treatment of subjects with metastatic EGFR C797S mutation-positive NSCLC as detected by an approved molecular test, whose disease has progressed on or after first-line osimertinib. In a specific embodiment, the Salt or Solid Form of the Disclosure, or a pharmaceutical composition disclosed herein, is used in combination with a 1^{st} or 3^{rd} generation TKI indicated for the treatment of subject with metastatic NSCLC.

In a particular embodiment, the deletions, mutations, and insertions disclosed herein are detected by an FDA-approved test.

A person of ordinary skill in the art can readily determine the certain EGFR alterations a subject possesses in a cell, cancer, gene, or gene product, e.g., whether a subject has one or more of the mutations or deletions described herein using a detection method selected from those known in the art such as hybridization-based methods, amplification-based methods, microarray analysis, flow cytometry analysis, DNA sequencing, next-generation sequencing (NGS), primer extension, PCR, in situ hybridization, fluorescent in situ hybridization, dot blot, and Southern blot.

To detect one or more EGFR deletions and/or mutations, a primary tumor sample, circulating tumor DNA (ctDNA), circulating tumor cells (CTC), and/or circulating exosomes may be collected from a subject. The samples are processed, the nucleic acids are isolated using techniques known in the art, then the nucleic acids are sequenced using methods known in the art. Sequences are then mapped to individual exons, and measures of transcriptional expression (such as RPKM, or reads per kilobase per million reads mapped), are quantified. Raw sequences and exon array data are available from sources such as TCGA, ICGC, and the NCBI Gene Expression Omnibus (GEO). For a given sample, individual exon coordinates are annotated with gene identifier information, and exons belonging to kinase domains are flagged. The exon levels are then z-score normalized across all tumors samples.

The Salts and Solid Forms of the Disclosure, or pharmaceutical compositions disclosed herein, may be used for treating to a subject who has become refractory to treatment with one or more other EGFR inhibitors. "Refractory" means that the subject's cancer previously responded to drugs but later responds poorly or not at all. In some some embodiments, the subject has become refractory to one or more first generation EGFR inhibitors such as erlotinib, gefitinib, icotinib or lapatinib. In some embodiments, the subject has been become refractory to treatment with one or more second generation EGFR inhibitors such as afatinib, dacomitinib, poziotinib, or neratinib. In some embodments the subject has become refractory to treatment with one or more first generation inhibitors and one or more second generation inhibitors. In some embodiments, the subject has become refractory to treatment with one or more third generation inhibitors such as osimertinib, nazartinib, or avitinib. In one embodiment, the subject has become refractory to treatment with one or more first generation EGFR inhibitors and one or more third generation EGFR inhibitors. In some embodiments, the subject has become refractory to treatment with one or more second generation EGFR inhibitors and one or more third generation EGFR inhibitors. In some embodiments, the subject has become refractory to treatment with one or more first generation inhibitors, and one or more third generation EGFR inhibitors.

### Combinations

The Salt or Solid Form of the Disclosure, or pharmaceutical compositions disclosed herein can be used in combination with one or more additional pharmacologically active substances. For example, the disclosure includes methods of treating a condition/disease/ or cancer comprising administering to a subject in need thereof a Salt or Solid Form of the Disclosure, or a pharmaceutical composition disclosed herein, in combination with an EGFR (or EGFR mutant) inhibitor, such as afatinib, osimertinib, lapatinib, erlotinib, dacomitinib, poziotinib, neratinib, gefitinib JBJ-04-125-02, alflutinib (AST 2818), almonertinib (HS10296), BBT-176, BI-4020, CH7233163, gilitertinib, JND-3229, lazertinib, nazartinib (EGF 816), PCC-0208027, rezivertinib (BPI-7711), TQB3804, zorifertinib (AZ-3759), or DZD9008; an EGFR antibody such as cetuximab, panitumumab, necitumumab, HLX07, JMT101; or a bispecific EGFR and MET antibody (*e.g.*, amivantamab ((JNJ-61186372, JNJ-372)). For the treatment of cancer *e.g.,* NSCLC using a Salt or Solid Form of the Disclosure, or a pharmaceutical composition disclosed herein, in combination with a first line therapy, for example a first, second, or third generation EGFR inhibitor (*i.e.,* as an initial treatment before the cancer has become refractory) may forestall or delay the cancer from becoming refractory. Typically, the cancer is characterized by one of the EGFR genotypes described herein.

Alternatively, a Salt or Solid Form of the Disclosure, or a pharmaceutical composition disclosed herein, can be administered in combination with other anti-cancer agents that are not EGFR inhibitors *e.g*., in combination with MEK, including mutant MEK inhibitors (trametinib, cobimtetinib, binimetinib, selumetinib, refametinib); c-MET, including mutant c-Met inhibitors (savolitinib, cabozantinib, foretinib, glumetinib, tepotinib) and MET antibodies (emibetuzumab, telisotuzumab vedotin (ABBV 339)); mitotic kinase inhibitors (CDK4/6 inhibitors such as palbociclib, ribociclib, abemacicilb, GIT38); anti-angiogenic agents e.g., bevacizumab, nintedanib; apoptosis inducers such as Bcl-2 inhibitors e.g, venetoclax, obatoclax, navitoclax, palcitoclax (APG-1252), and Mcl-1 inhibitors *e.g*., AZD-5991, AMG-176, S-64315; mTOR inhibitors e.g, rapamycin, temsirolimus, everolimus, ridoforolimus; RET inhibitors, like pralsetinib and selpercatinib, and PI3K inhibitors dactolisib (BEZ235), pictilisib (GDC-0941), LY294002, idelalisib (CAL-101); JAK inhibitors (*e.g.,* AZD4205, itacitinib), Aurora A inhibitors (*e.g.,* alisertib); BCR/ABL and/or Src family tyrosine kinase inhibitors (*e.g.,* dasatinib); VEGF inhibitors (*e.g.,* MP0250; ramucirumab); multi-kinase protein inhibitors (*e.g*., anlotinib, midostaurin); PARP inhibitors (*e.g.,* niraparib); platinum therapies (*e.g.,* cisplatin (CDDP), carboplatin (CBDCA), or nedaplatin (CDGP)); PD-L1 inhibitors (*e.g*., durvalumab (MEDI 4736)); HER2/neu receptor inhibitors (*e.g.,* trastuzumab); anti-HER2 or anti-HER3 antibody-drug conjugates (*e.g.,* patritumab deruxtecan (U3-1402), trastuzumab emtansine); or immunogene therapy (*e.g.,* oncoprex).

A "subject" is a human in need of treatment.

### Methods of Administration and Dosage Forms

The precise amount of the Salt or Solid Form of the Disclosure, or a pharmaceutical composition disclosed herein, administered to provide an "effective amount" to the subject will depend on the mode of administration, the type, and severity of the cancer, and on the characteristics of the subject, such as general health, age, sex, body weight, and tolerance to drugs. The skilled artisan will be able to determine appropriate dosages depending on these and other factors. When administered in combination with other therapeutic agents, *e.g.,* when administered in combination with an anti-cancer agent, an "effective amount" of any additional therapeutic agent(s) will depend on the type of drug used. Suitable dosages are known for approved therapeutic agents and can be adjusted by the skilled artisan according to the condition of the subject, the type of condition(s) being treated and the amount of a Salt or Solid Form of the Disclosure being used by following, for example, dosages reported in the literature and recommended in the Physician's Desk Reference (57th Ed., 2003).

"Treating" or "treatment" refers to obtaining a desired pharmacological and/or physiological effect. The effect can be therapeutic, which includes achieving, partially or substantially, one or more of the following results: partially or substantially reducing the extent of the disease, condition or cancer; ameliorating or improving a clinical symptom or indicator associated with the disease, condition or cancer; delaying, inhibiting or decreasing the likelihood of the progression of the disease, condition or cancer; or decreasing the likelihood of recurrence of the disease, condition or cancer.

The term "effective amount" means an amount when administered to the subject which results in beneficial or desired results, including clinical results, e.g., inhibits, suppresses or reduces the symptoms of the condition being treated in the subject as compared to a control. For example, a therapeutically effective amount can be given in unit dosage form *(e.g.,* 0.1 mg to about 50 g per day, alternatively from 1 mg to about 5 grams per day; and in another alternatively from 10 mg to 1 gram per day).

The terms "administer", "administering", "administration", and the like, as used herein, refer to methods that may be used to enable delivery of compositions to the desired site of biological action. These methods include, but are not limited to, intraarticular (in the joints), intravenous, intramuscular, intratumoral, intradermal, intraperitoneal, subcutaneous, orally, topically, intrathecally, inhalationally, transdermally, rectally, and the like. Administration techniques that can be employed with the agents and methods described herein are found in *e.g.,* Goodman and Gilman, The Pharmacological Basis of Therapeutics, current ed.; Pergamon; and Remington's, Pharmaceutical Sciences (current edition), Mack Publishing Co., Easton, Pa.

In addition, a Salt or Solid Form of the Disclosure, or a pharmaceutical composition disclosed herein, can be co-administered with other therapeutic agents. As used herein, the terms "co-administration", "administered in combination with", and their grammatical equivalents, are meant to encompass administration of two or more therapeutic agents to a single subject, and are intended to include treatment regimens in which the agents are administered by the same or different route of administration or at the same or different times. In some embodiments the Salt or Solid Form of the Disclosure, or a pharmaceutical composition disclosed herein, will be co-administered with other agents. These terms encompass administration of two or more agents to the subject so that both agents and/or their metabolites are present in the subject at the same time. They include simultaneous administration in separate compositions, administration at different times in separate compositions, and/or administration in a composition in which both agents are present. Thus, in some embodiments, the Salt or Solid Form of the Disclosure, or a pharmaceutical composition disclosed herein, and the other agent(s) are administered in a single composition. In some embodiments, the compounds described herein and the other agent(s) are admixed in the composition.

The particular mode of administration and the dosage regimen will be selected by the attending clinician, taking into account the particulars of the case (*e.g.* the subject, the disease, the disease state involved, the particular treatment). Treatment can involve daily or multi-daily or less than daily (such as weekly or monthly etc.) doses over a period of a few days to months, or even years. However, a person of ordinary skill in the art would immediately recognize appropriate and/or equivalent doses looking at dosages of approved compositions for treating a disease using the disclosed EGFR inhibitors for guidance.

The Salt or Solid Form of the Disclosure, or a pharmaceutical composition disclosed herein, can be administered to a patient in a variety of forms depending on the selected route of administration, as will be understood by those skilled in the art. The Salt or Solid Form of the Disclosure, or a pharmaceutical composition disclosed herein, may be administered, for example, by oral, parenteral, buccal, sublingual, nasal, rectal, patch, pump or transdermal administration and the pharmaceutical compositions formulated accordingly. Parenteral administration includes intravenous, intraperitoneal, subcutaneous, intramuscular, transepithelial, nasal, intrapulmonary, intrathecal, rectal and topical modes of administration. Parenteral administration can be by continuous infusion over a selected period of time.

The pharmaceutical composition of the disclosure is formulated to be compatible with its intended route of administration. In an embodiment, the composition is formulated in accordance with routine procedures as a pharmaceutical composition adapted for intravenous, subcutaneous, intramuscular, oral, intranasal, or topical administration to human beings. In preferred embodiments, the pharmaceutical composition is formulated for intravenous administration.

Typically, for oral therapeutic administration, a Salt or Solid Form of the Disclosure may be incorporated with excipient and used in the form of ingestible tablets, buccal tablets, troches, capsules, elixirs, suspensions, syrups, wafers, and the like.

Typically for parenteral administration, solutions of a Salt or Solid Form of the Disclosure be prepared in water suitably mixed with a surfactant such as hydroxypropylcellulose. Dispersions can also be prepared in glycerol, liquid polyethylene glycols, DMSO and mixtures thereof with or without alcohol, and in oils. Under ordinary conditions of storage and use, these preparations contain a preservative to prevent the growth of microorganisms.

Typically, for injectable use, sterile aqueous solutions or dispersion of, and sterile powders of, a Salt or Solid Form of the Disclosure for the extemporaneous preparation of sterile injectable solutions or dispersions are appropriate.

### EXPERIMENTAL

### Abbreviations:

| **Abbreviation** | **Solvent** | **Abbreviation** | **Solvent** |
|---|---|---|---|
| ACN | Acetonitrile | MeOH | Methanol |
| IPA | 2-Propanol | MeOAc | Methyl Acetate |
| ACN | Acetonitrile | MtBE | tert-Butyl Methyl Ether |
| DCM | Dichloromethane | THF | Tetrahydrofuran |
| EtOH | Ethanol | TFE | Trifluoroethanol |
| EtOAc | Ethyl Acetate | 2-MeTHF | 2-Methyltetrahydrofuran |
| MEK | Methyl Ethyl Ketone | n-PA | n-Propanol |
| TFA | Trifluoroacetic Acid | DMSO | Dimethyl sulfoxide |
| TEA | Triethylamine | DMF | Dimethylformamide |
| PE | Petroleum ether | DMA/DMAc | Dimethylacetamide |
| IPOAc/IPAc | Isopropyl acetate | FaSSGF | Fasted State Simulated Gastric Fluid |
| FaSSIF | Fasted State Simulated Intestinal Fluid | FeSSIF | Fed State Simulated Intestinal Fluid |

| **Instruments** | |
|---|---|
| **Full Name** | **Abbreviation** |
| Differential scanning calorimetry | DSC |
| Dynamic Vapor Sorption | DVS |
| High Performance Liquid Chromatography | HPLC |
| Nuclear Magnetic Resonance | NMR |
| X-ray Powder Diffraction | XRPD |
| Thermogravimetric Analysis | TGA |

| **Units** | |
|---|---|
| **Full Name** | **Abbreviation** |
| Celsius | C |
| Degrees | ° |
| Equivalents | eq. |
| Gram | g |
| Hour | h |
| Kelvin | K |
| Liters | L |
| Milligrams | mg |
| Milliliters | mL |
| Minute | min |
| Milliamp | mA |
| Kilovolt | kV |
| Relative Humidity | RH |
| Room temperature | RT |
| Second | sec |
| volume | vol. |
| Volume ratio | v/v |
| Watt | W |
| Weight | wt. |
| Weight Percentage | wt.% |

### Analysis Conditions

### Differential Scanning Calorimetry (DSC)

DSC was performed using a Mettler Toledo DSC3⁺. The sample (1-5 mg) was weighed directly in a 40 µL hermetic aluminum pan with pin-hole and analyzed according to the parameters below:

| **Parameters** | |
|---|---|
| Method | ***Ramp*** |
| Sample size | 3-5 mg |
| Heating rate | 10.0 °C/min |
| Temperature range | 30 to 300 °C |
| Method gas | N₂ at 60.00 mL/min |

| **Parameters** | |
|---|---|
| Method | ***Modulation*** |
| Sample size | 5-10 mg |
| Amplitude | 1 °C |
| Period | 60 s |
| Heating rate | 2.0 °C/min |
| Temperature range | 30 to 300 °C |
| Method gas | N₂ at 60.00 mL/min |

### Dynamic Vapor Sorption (DVS)

DVS was performed using a DVS Intrinsic 1. The sample (5-25 mg) was loaded into a sample pan, suspended from a microbalance and exposed to a humidified stream of nitrogen gas. The sample was held for a minimum of 5 min at each level and only progressed to the next humidity level if there was < 0.002 % change in weight between measurements (interval: 60 s) or 60 min had elapsed. The following program was used:
1- Equilibration at 50 % RH
2- 50 % to 2 %. (50 %, 40 %, 30 %, 20 %, 10 %, and 2%)
3- 2 % to 95 % (2 %, 10 %, 20 %, 30 %, 40 %, 50 %, 60 %, 70 %, 80 %, 90 %, 95 %)
4- 95 % to 2 % (95 %, 80 %, 70 %, 60 %, 50 %, 40 %, 30 %, 20 %, 10 %, 2 %)
5- 2 % to 50 % (2 %, 10 %, 20 %, 30 %, 40 %, 50 %)

### High Performance Liquid Chromatography (HPLC)

HPLC was conducted using an Agilent 1220 Infinity LC. Flow rate range was 0.2-5.0 mL/min, operating pressure range was 0-600 bar, temperature range was 5 °C above ambient to 60 °C, and wavelength range was 190-600 nm. The HPLC method is shown below:

| **Parameters** | | | |
|---|---|---|---|
| Mobile Phase A | 0.05% TFA in distilled water | | |
| Mobile Phase B | 0.05% TFA in ACN | | |
| Diluent | ACN:water (1:1 vol) | | |
| Injection Volume | 5 µL | | |
| Monitoring Wavelength | 275 nm | | |
| Column | Waters Acquity UPLC BEH C-18, 2.1 × 50 mm, 1.7 µm | | |
| Column Temperature | 40 °C | | |

| | **Time (min)** | **%A** | **Flow Rate (mL/min)** |
|---|---|---|---|
| Gradient Method | 0 | 95 | 0.8 |
| | 6 | 5 | 0.8 |
| | 8 | 5 | 0.8 |
| | 8.5 | 95 | 0.8 |
| | 10 | 95 | 0.8 |

### Liquid Chromatography-Mass Spectrometry (LCMS)

The liquid chromatography-mass spectrometry (LC-MS) data were obtained with an Agilent model-1260 LC system using an Agilent model 6120 mass spectrometer utilizing ES-API ionization fitted with an Agilent Poroshel 120 (EC-C18, 2.7 µm particle size, 3.0 x 50mm dimensions) reverse-phase column at 22.4 °C. The mobile phase consisted of a mixture of solvent 0.1% formic acid in water and 0.1% formic acid in acetonitrile. A constant gradient from 95% aqueous/5% organic to 5% aqueous/95% organic mobile phase over the course of 4 minutes was utilized. The flow rate was constant at 1 mL/min.

### Nuclear Magnetic Resonance (NMR)

Proton NMR (¹H NMR) was performed on a Bruker Avance 300 MHz spectrometer. Solids were dissolved in 0.75 mL deuterated solvent in a 4 mL vial, transferred to an NMR tube (Wilmad 5mm thin wall 8" 200MHz, 506-PP-8) and analyzed according to the following parameters:

| **Parameters - Bruker Avance 300** | |
|---|---|
| Instrument | Bruker Avance 300 MHz spectrometer |
| Temperature | 300 K |
| Probe | 5 mm PABBO BB-1H/DZ-GRD Z104275/0170 |
| Number of scans | 16 |
| Relaxation delay | 1.000 s |
| Pulse width | 14.2500 µs |
| Acquisition time | 2.9999 s |
| Spectrometer frequency | 300.15 Hz |
| Nucleus | ¹H |

### pH Measurement

pH was measured using a Mettler Toledo FP20 bench meter equipped with a Mettler Toledo InLab Micro pH electrode. The electrode had a ceramic junction and membrane resistance of < 600 MΩ. The internal reference electrolyte solution used was KCl and the operating range was 0-14 pH units and 0-80 °C.

### Thermogravimetric Analysis and Differential Scanning Calorimetry (TGA & DSC)

TGA and DSC were performed on the same sample simultaneously using a Mettler Toledo TGA/DSC³⁺. Protective and purge gas was nitrogen at flowrate 20-30 mL/min and 50-100 mL/min respectively. The desired amount of sample (5-10 mg) was weighed directly in ahermetic aluminum pan with pin-hole and analyzed according to the parameters below:

| **Parameters** | |
|---|---|
| Method | Ramp |
| Sample size | 5-10 mg |
| Heating rate | 10.0 °C/min |
| Temperature range | 30 to 300 °C |

### X-Ray Powder Diffraction (XRPD)

XRPD was performed using a Bruker D8 Advance equipped with LYNXEYE detector in reflection mode (*i.e.* Bragg-Brentano geometry). Samples were prepared on Si zero-return wafers. The parameters for XRPD methods used are listed below:

| **Parameters for Reflection Mode for Regular Scans** | |
|---|---|
| X-ray wavelength | Cu Kα1, 1.540598 Å, |
| X-ray tube setting | 40 kV, 40 mA |
| Slit condition | 0.6 mm div. + 2.5° soller |
| Scan mode | Step |
| Scan range (°2θ) | 4 - 30 |
| Step size (°2θ) | 0.03 |
| Dwell time (s/step) | 0.23 |
| Spin | Yes (0.5 Hz) |

| **Parameters for Reflection Mode for High Resolution Scans** | |
|---|---|
| X-ray wavelength | Cu Kα1, 1.540598 Å, |
| X-ray tube setting | 40 kV, 15 mA |
| Slit condition | 1.25 ° div., Ni kβ filter, 0.3 mm rec. |
| Scan mode | Continuous |
| Scan range (°2θ) | 4 - 40 |
| Step size (°2θ) | 0.05 |
| Dwell time (s/step) | 1.25 |
| Spin | No |

### Example 1: Preparations and Characterization of Crystalline Form of 1:0.5 Compound (I) Semi-Succinate Form C

### 1.1 Preparation

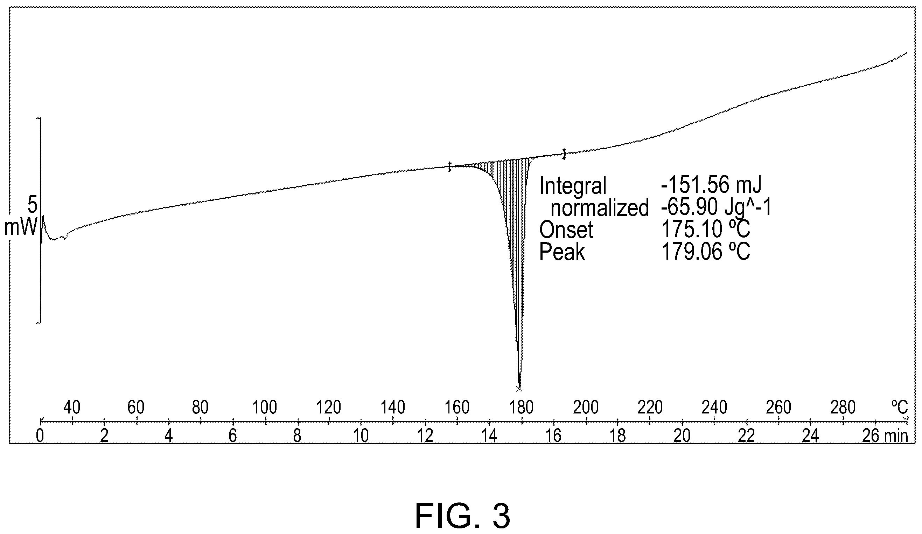

Compound (I) in freebase (301 mg) was weighed in a 20 mL vial, to which 1.3 eq of succinic acid (83.4 mg) and a stir bar were added. 15 volumes of EtOAc (4.51 mL) were added at 45 °C and left to stir for one hour. Solids were bright yellow and were filtered and washed with 2 x 2 vol. of EtOAc. Solids were dried in a vacuum oven at 50 °C overnight. The XRPD pattern indicated excess succinic acid was present. Solids were then re-slurried in 5 vol.
(1.36 mL) of IPA for 1 hour and were then filtered and washed with 2 × 2 vol of IPA. Solids were dried in a vacuum oven at 50°C overnight. Purity by HPLC was 99.50 area %. The solid obtained was further characterized by XRPD using the Regular Scan Method (see Figure 1 and Table 1), TGA-DSC (Figures 2 and 3), and DVS. It was determined that the counter-ion stoichiometry (API:CI) is 1:0.5.

The combined DSC and TGA thermogram showed a total mass loss of 0.85 wt.% and an endotherm onset at 176.3 °C (Figure 2). The DSC alone showed an endotherm onset at 175.1 °C (Figure 3).

**Table 1. Peak list for XRPD pattern of 1:0.5 Compound (I) Semi-Succinate**

| **2θ (deg)** | **d-Spacing (ang.)** | **Relative Intensity (%)** |
|---|---|---|
| 4.53 | 19.50 | 100 |
| 6.25 | 14.13 | 5 |
| 6.73 | 13.13 | 16 |
| 8.94 | 9.88 | 68 |
| 9.26 | 9.54 | 95 |
| 11.05 | 8.00 | 20 |
| 11.90 | 7.43 | 15 |
| 12.27 | 7.21 | 28 |
| 12.97 | 6.82 | 38 |
| 13.36 | 6.62 | 7 |
| 14.36 | 6.16 | 16 |
| 14.99 | 5.90 | 8 |
| 15.32 | 5.78 | 99 |
| 15.59 | 5.68 | 10 |
| 16.30 | 5.44 | 16 |
| 16.81 | 5.27 | 30 |
| 17.81 | 4.98 | 50 |
| 18.11 | 4.89 | 36 |
| 18.45 | 4.81 | 32 |
| 18.95 | 4.68 | 11 |
| 20.48 | 4.33 | 22 |
| 21.28 | 4.17 | 11 |
| 21.60 | 4.11 | 12 |
| 22.28 | 3.99 | 32 |
| 22.93 | 3.88 | 11 |
| 25.98 | 3.43 | 21 |
| 28.59 | 3.12 | 6 |
| 29.05 | 3.07 | 5 |
| 29.55 | 3.02 | 5 |

### 1.2 DVS of Crystalline Form of 1:0.5 Compound (I) Semi-Succinate

DVS was completed and showed a mass change of 7.1 wt. % between 2 and 95% relative humidity at 25 °C. After the standard 60 minutes at 95% RH, the compound had not reached equilibrium and thus this interval was held for a total of 240 minutes. After this time the compound still had not reached equilibrium, and experiment was continued as described in the DVS analysis conditions above.

### Example 2: Preparations and Characterization of Crystalline Form of 1:0.5 Compound (I) Semi-Glutarate Form D

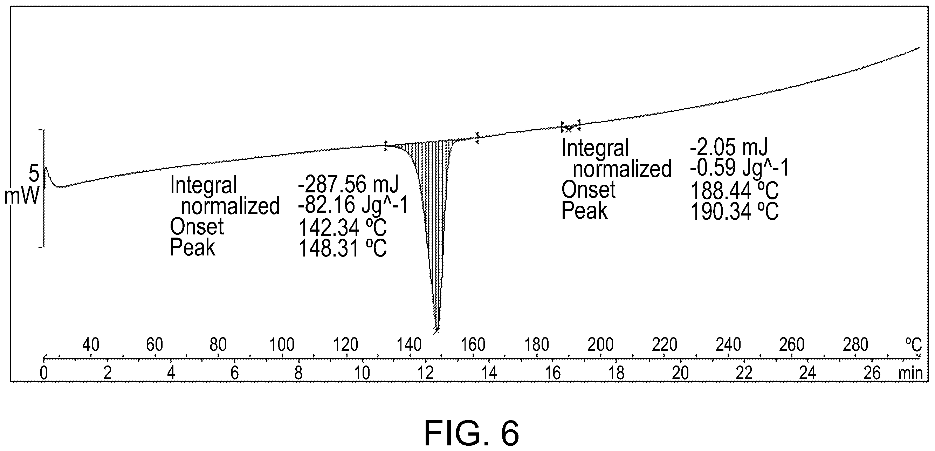

Compound (I) in freebase (401 mg) was weighed in a 20 mL vial, to which 1.1 eq of glutaric acid (104.6 mg) and a stir bar were added. 15 volumes of EtOAc were added at 45 °C. After one hour, the temperature was decreased to RT, and the thin yellow slurry was left to stir overnight. The following morning, the slurry was noticeably thicker and a more vibrant yellow in color. The slurry was filtered, washed with 2 x 2 volumes of EtOAc, and dried under active vacuum (-30 in Hg) for 5 h at 50 °C. Purity by HPLC was 99.45 area % and stoichiometry was calculated to be 1:0.53 Compound (I) Semi-Glutarate based on ¹H NMR. The solid obtained was further characterized by XRPD using the Regular Scan Method (see Figure 4 and Table 2) and TGA-DSC (Figures 5 and 6).

The combined DSC and TGA thermogram showed essentially no mass loss and an endotherm onset at 142.5 °C (Figure 5). The DSC alone showed a n endothermic event with an onset of 142.3 °C, and a small endotherm (-0.59 J/g) with an onset of 188.4 °C (Figure 6).

**Table 2. Peak list for XRPD pattern of 1:0.5 Compound (I) Semi-Glutarate**

| **2θ (deg)** | **d-Spacing (ang.)** | **Relative Intensity (%)** |
|---|---|---|
| 6.58 | 13.43 | 12 |
| 7.42 | 11.90 | 14 |
| 8.84 | 10.00 | 100 |
| 10.52 | 8.40 | 5 |
| 12.27 | 7.21 | 25 |
| 12.89 | 6.86 | 12 |
| 14.78 | 5.99 | 27 |
| 16.12 | 5.49 | 81 |
| 16.57 | 5.35 | 5 |
| 18.30 | 4.84 | 46 |
| 18.65 | 4.75 | 33 |
| 19.23 | 4.61 | 10 |
| 20.03 | 4.43 | 13 |
| 20.45 | 4.34 | 11 |
| 22.20 | 4.00 | 13 |
| 22.81 | 3.90 | 11 |
| 24.74 | 3.60 | 6 |

### Example 3: Preparations and Characterization of Crystalline Form of 1:1 Compound (I) Fumarate Form E

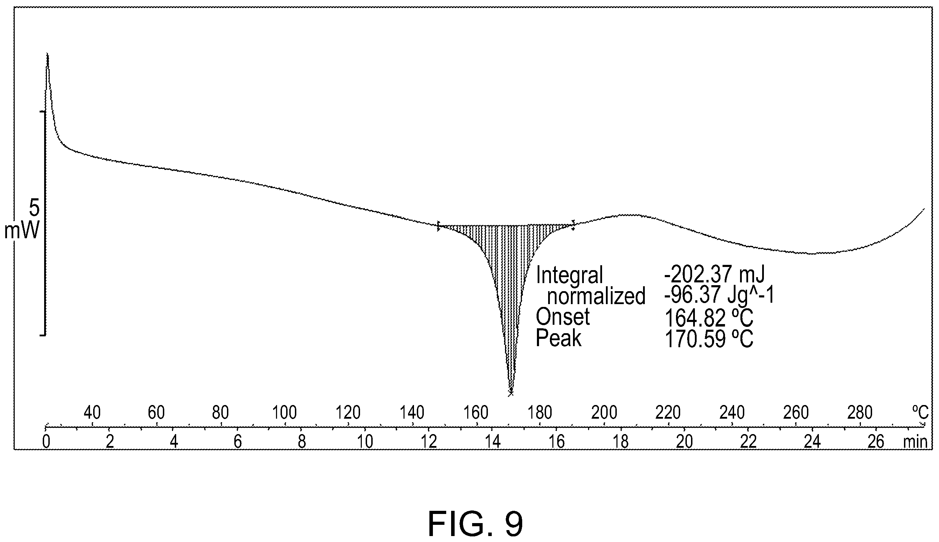

Compound (I) in freebase (409.5 mg) was weighed in a 20 mL vial, to which 1.1 eq of fumaric acid (96.6 mg) and a stir bar were added. 15 volumes of EtOAc were added at 45 °C. After one hour, the temperature was decreased to RT, and the slurry remained pastel-yellow and thin after stirring overnight. TFE was added (100 µL) to aid in the dissolution of the solids at 45 °C. The solvent was evaporated with stirring at 45 °C and 5 mL of TFE was added to achieve full dissolution of the solids. The solvent was once again evaporated with active stirring overnight, and the vial was placed under active vacuum at 50 °C for 3 h. Once dry, 15 vol. of EtOAc was added to the solids at 45 °C. The bright-yellow slurry was stirred at 45 °C for one hour, then at RT. The slurry was filtered, washed with 2 x 2 volumes of EtOAc, and dried under active vacuum (-30 in Hg) for 5 h at 50 °C. Purity by HPLC was 99.31 area % and the stoichiometry was calculated to be 1: 0.95 Compound (I) Fumarate based on ¹H NMR. The solid obtained was further characterized by XRPD using the Regular Scan Method (see Figure 7 and Table 3) and TGA-DSC (Figures 8 and 9).

The combined DSC and TGA thermogram showed a total mass loss of 0.3 wt.% and an endotherm onset at 162.2 °C (Figure 8). The DSC alone showed an endotherm onset at 164.8 °C (Figure 9).

**Table 3. Peak list for XRPD pattern of 1:1 Compound (I) Fumarate**

| **2θ (deg)** | **d-Spacing (ang.)** | **Relative Intensity (%)** |
|---|---|---|
| 5.11 | 17.27 | 11 |
| 6.33 | 13.95 | 88 |
| 7.89 | 11.19 | 11 |
| 7.97 | 11.09 | 9 |
| 8.50 | 10.40 | 100 |
| 9.02 | 9.79 | 50 |
| 9.88 | 8.95 | 28 |
| 10.17 | 8.69 | 10 |
| 10.65 | 8.30 | 12 |
| 11.45 | 7.72 | 22 |
| 11.45 | 7.72 | 22 |
| 11.79 | 7.50 | 8 |
| 12.09 | 7.31 | 45 |
| 12.26 | 7.21 | 19 |
| 12.59 | 7.03 | 28 |
| 12.84 | 6.89 | 10 |
| 13.40 | 6.60 | 12 |
| 13.51 | 6.55 | 23 |
| 13.74 | 6.44 | 5 |
| 13.79 | 6.42 | 5 |
| 14.47 | 6.12 | 74 |
| 14.73 | 6.01 | 23 |
| 14.83 | 5.97 | 17 |
| 15.11 | 5.86 | 30 |
| 15.22 | 5.82 | 29 |
| 15.42 | 5.74 | 33 |
| 15.73 | 5.63 | 63 |
| 15.96 | 5.55 | 27 |
| 16.29 | 5.44 | 27 |
| 16.74 | 5.29 | 25 |
| 17.72 | 5.00 | 10 |
| 18.03 | 4.92 | 50 |
| 18.17 | 4.88 | 31 |
| 18.28 | 4.85 | 24 |
| 18.87 | 4.70 | 36 |
| 18.87 | 4.70 | 36 |
| 19.32 | 4.59 | 34 |
| 19.66 | 4.51 | 49 |
| 19.81 | 4.48 | 15 |
| 20.13 | 4.41 | 37 |
| 20.34 | 4.36 | 20 |
| 20.62 | 4.30 | 33 |
| 20.68 | 4.29 | 34 |
| 20.94 | 4.24 | 14 |
| 21.32 | 4.16 | 30 |
| 21.45 | 4.14 | 20 |
| 21.66 | 4.10 | 17 |
| 21.93 | 4.05 | 37 |
| 22.13 | 4.01 | 18 |
| 22.38 | 3.97 | 14 |
| 22.59 | 3.93 | 25 |
| 22.71 | 3.91 | 13 |
| 22.96 | 3.87 | 11 |
| 23.16 | 3.84 | 30 |
| 23.34 | 3.81 | 27 |
| 23.66 | 3.76 | 17 |
| 23.81 | 3.73 | 18 |
| 24.11 | 3.69 | 44 |
| 24.24 | 3.67 | 15 |
| 24.77 | 3.59 | 36 |
| 25.15 | 3.54 | 7 |
| 25.19 | 3.53 | 8 |
| 25.46 | 3.50 | 17 |
| 25.70 | 3.46 | 19 |
| 25.74 | 3.46 | 18 |
| 26.27 | 3.39 | 17 |
| 26.71 | 3.33 | 18 |
| 27.15 | 3.28 | 10 |
| 27.55 | 3.23 | 8 |
| 29.16 | 3.06 | 10 |
| 29.16 | 3.06 | 10 |

### Example 4: Preparations and Characterization of Crystalline Form A of Compound (I) Free Base

### 4.1 Synthesis of N-(2-((3S,4R)-3-fluoro-4-methoxypiperidin-1-yl)pyrimidin-4-yl)-5-isopropyl-8-((2R,3S)-2-methyl-3-((methylsulfonyl)methyl)azetidin-1-yl)isoquinolin-3-amine [Compound (I)]

### 4.1.1 Synthesis of (2R,3S)-2-methyl-3-(methylsulfonylmethyl)azetidine:

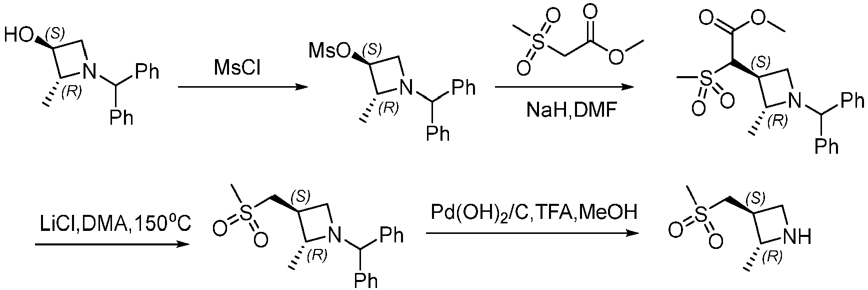

### Step 1: Synthesis of (2R,3S)-1-benzhydryl-2-methylazetidin-3-yl methanesulfonate:

(2R,3S)-1-benzhydryl-2-methylazetidin-3-ol (Pharmablock, 20 g, 78.9 mmol) was dissolved in 300 mL DCM and TEA (9.55 g, 94.6 mmol) was added and the reaction mixture cooled in an ice bath. Mesyl chloride (9.93g, 86.7mmol) was added dropwise and allowed to stir, warming slowly to rt and stirred overnight. The mixture was diluted with DCM and washed with water and the organic phase dried over sodium sulfate, filtered and evaporated to give 26 g (98%) of the title compound as a viscous yellow oil.
Analytical Data: LC-MS: (ES, m/z) = 332 [M+1].

### Step 2: Synthesis of (S)-methyl 2-((2R,3S)-1-benzhydryl-2-methylazetidin-3-yl)-2-(methylsulfonyl) acetate:

(2R,3S)-1-benzhydryl-2-methylazetidin-3-yl methanesulfonate (26 g, 78.4 mmol) and methyl 2-(methylsulfonyl)acetate (15.3 g, 101 mmol) were dissolved in 260 mL DMF and then NaH (3.75 g of 60% dispersion in mineral oil, 6.63 mmol) was added and stirred for ~15 minutes, until hydrogen evolution had ceased. The reaction mixture was heated to 80 °C overnight. The reaction was cooled and then diluted with ~200 mL water and extracted with EtOAc and combined organics washed with water, brine and dried over sodium sulfate, filtered and evaporated to give the crude product. The residue was purified by chromatography (0 to 7% MeOH/DCM). Pure fractions combined and evaporated to give 24 g (80%) of the title compound as a pale-yellow foam.

### Step 3: Synthesis of (2R,3S)-1-benzhydryl-2-methyl-3-(methylsulfonylmethyl)azetidine:

(S)-methyl-2-((2R,3S)-1-benzhydryl-2-methylazetidin-3-yl)-2-(methylsulfonyl)acetate (24 g, 61.9 mmol) was dissolved in 240 mL DMA and lithium chloride (20.9 g, 495 mmol) was added and the flask put into a preheated block that was kept at 150 °C. LC/MS indicated the starting material was consumed after 1.5 h. Cooled to room temperature and dilute with water, extracted with EtOAc and the combined organics washed with water, brine and dried over sodium sulfate. Filtered and evaporated to give the crude product and further purified by chromatography (0 to 5% MeOH/DCM). Pure fractions were combined and evaporated to give 19 g (93%) of the title compound as a pale-yellow foam.
Analytical Data: LC-MS: (ES, m/z) = 330 [M+1].

### Step 4: Synthesis of (2R,3S)-2-methyl-3-(methylsulfonylmethyl)azetidine:

To a solution of (2R,3S)-1-(diphenylmethyl)-3-(methanesulfonylmethyl)-2-methylazetidine (1 9g, 57.3 mmol) in MeOH (270 mL) was added TFA (9 mL) and Pd(OH)₂ (5.7 g), the reaction was stirred overnight at rt under H₂ atmosphere. The reaction mixture was filtered and evaporated to give the crude title compound (17 g) as a light-brown oil.
Analytical Data: LC-MS: (ES, m/z) = 164 [M+1].

### 4.1.2 Synthesis of 2-((3S,4R)-3-fluoro-4-methoxypiperidin-1-yl)pyrimidin-4-amine

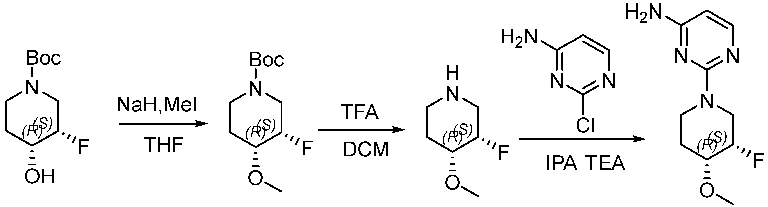

### Step 1: Synthesis of (3S,4R)-tert-butyl 3-fluoro-4-methoxypiperidine-1-carboxylate:

Sodium hydride (218.90 mg, 9.122 mmol, 4 equiv.) was added to tert-butyl (3S,4R)-3-fluoro-4-hydroxypiperidine-1-carboxylate (500 mg, 2.280 mmol, 1 equiv.) in THF (10 mL) at 0 °C. After stirring for 20 min, methyl iodide (1294.73 mg, 9.122 mmol, 4 equiv.) was added. The resulting solution was stirred for additional 1 h at 0 °C. The reaction was then quenched by addition of 10 mL of water. The solids were filtered out. The resulting solution was extracted with EtOAc and concentrated under vacuum. This resulted in 500 mg (94.1%) of the title compound as light-yellow oil.
Analytical Data: LC-MS: (ES, m/z) = 178 [M+1-56].

### Step 2: Synthesis of (3S,4R)-3-fluoro-4-methoxypiperidine:

The solution of tert-butyl (3S,4R)-3-fluoro-4-methoxypiperidine-1-carboxylate (500 mg, 2.143 mmol, 1 equiv.) in TFA/DCM (3/10 mL) was stirred for 1 h at rt. The resulting mixture was concentrated under vacuum to afford 500 mg (crude) of the title compound as a solid.

### Step 3: Synthesis of 2-((3S,4R)-3-fluoro-4-methoxypiperidin-1-yl)pyrimidin-4-amine:

The mixture of (3S,4R)-3-fluoro-4-methoxypiperidine(3 g, 22.528 mmol, 1 equiv.), 2-chloropyrimidin-4-amine (2.33 g, 0.018 mmol, 0.8 equiv.) and TEA (6.84 g, 0.068 mmol, 3 equiv.) in IPA (3 mL) was stirred for 12 h at 100 °C. The solvent was removed under vacuum and residue was purified by FLASH (5% MeOH in DCM) to give 3.3 g (66 %) of the title compound as a light-yellow solid.
Analytical Data: LC-MS: (ES, m/z) = 227 [M+1]. ¹H-NMR (400 MHz, 6d-DMSO) δ ppm 7.72 (d, 1H, J=5.6 Hz), 6.39 (s, 2H), 5.71 (d, 1H, J=5.6 Hz), 4.83 (d, 1H, J=49.3 Hz), 4.60 - 4.49 (m, 1H), 4.29 (d, 1H, J=13.3 Hz), 3.55 - 3.42 (m, 1H), 3.28 (d, 1H, J=13.3 Hz), 3.20 - 3.04 (m, 1H), 1.76 - 1.48 (m, 2H)

### 4.1.3 Synthesis of 8-bromo-3-chloro-5-isopropylisoquinoline

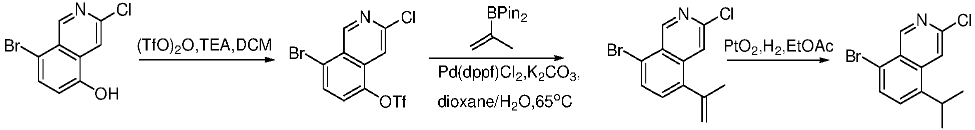

### Step 1: Synthesis of 8-bromo-3-chloroisoquinolin-5-yl trifluoromethanesulfonate:

Trifluoromethanesulfonyl trifluoromethanesulfonate (45.7 g, 162 mmol) was added dropwise to 8-bromo-3-chloroisoquinolin-5-ol (14 g, 54.1 mmol) and TEA (21.8 g, 216 mmol) in DCM (400 mL) at -60 °C. The resulting mixture was warmed to room temperature naturally and stirred at rt for 1h. The mixture was concentrated under vacuum. The residue was purified by a silica gel column with PE:EA=5:1 to afford 18 g (85%) the title compound as a white solid.
Analytical Data: LC-MS: (ES, *m*/*z*) = 392 [M+1]; 1H NMR (400 MHz, DMSO-d6) δ 9.46 (d, 1H, J = 0.8 Hz), 8.20 (d, 1H, J = 8.3 Hz), 8.02 (d, 1H, J = 8.4 Hz), 7.93 (d, 1H, J = 0.7 Hz).

### Step 2: Synthesis of 8-bromo-3-chloro-5-(prop-1-en-2-yl)isoquinoline:

The mixture of K₂CO₃ (6 g, 43.5 mmol), 8-bromo-3-chloroisoquinolin-5-yl trifluoromethanesulfonate (17 g, 43.5 mmol), 4,4,5,5-tetramethyl-2-(prop-1-en-2-yl)-1,3,2-dioxaborolane (7.30 g, 43.5 mmol) and Pd(dppf)Cl₂.CH₂Cl₂ (2.83 g, 3.48 mmol) in dioxane/H₂O (200/20 mL) was stirred for 3h at 45 °C. The mixture was diluted with 500 mL of EA and washed with brine 200mL*2. The organic layer was dried with Na₂SO₄ and concentrated under vacuum. The residue was purified by a silica gel column with PE:EtOAc = 20:1 to afford 8.0 g (67%) the title compound as an off-white solid.
Analytical Data: LC-MS: (ES, *m*/*z*) = 282 [M+1].

### Step 3: Synthesis of 8-bromo-3-chloro-5-isopropylisoquinoline:

PtO₂ (1.7 g 7.04 mmol) and 8-bromo-3-chloro-5-(prop-1-en-2-yl)isoquinoline (7.1 g, 25.1 mmol) in EA (300 mL) were stirred under an atmosphere of H₂ balloon at rt and stirred for 1h. The solid was filtered out. The mother solvent was concentrated under vacuum. The crude product was purified by a silica gel column with PE:EtOAc=10:1 to get 6.7 g (93%) the title compound as a brown solid.
Analytical Data: LC-MS: (ES, *m*/*z*) = 284 [M+1].

### 4.1.4 Synthesis of 3-chloro-5-isopropyl-8-((2R,3S)-2-methyl-3-((methylsulfonyl)methyl)azetidin-1-yl)isoquinoline

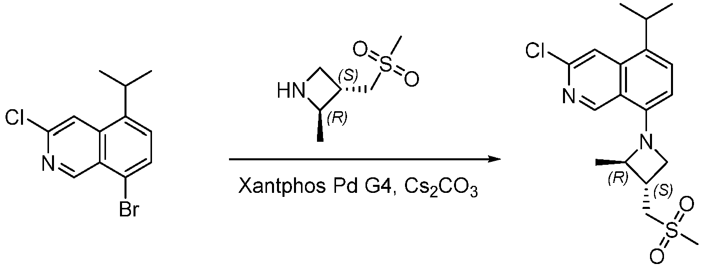

To a solution of 8-bromo-3-chloro-5-(propan-2-yl)isoquinoline (9 g, 31.6 mmol) in 1,4-dioxane (130 mL) was added (2R,3S)-3-(methanesulfonylmethyl)-2-methylazetidine (5.15 g, 31.6 mmol,), Cs₂CO₃ (20.6 g, 63.2 mmol) and Xantphos Pd G4 (1.51 g, 1.58 mmol) under nitrogen. The mixture was stirred at 100 °C for 3 h under nitrogen. The reaction mixture was cooled to rt and diluted with 300 mL of water. The resulting solution was extracted with EtOAc, washed with brine, dried over anhydrous sodium sulfate and concentrated under vacuum. The crude product was purified by silica gel chromatography (0-60% EtOAc in PE) to give 7.2 g (62.6%) of 3-chloro-8-[(2R,3S)-3-(methanesulfonylmethyl)-2-methylazetidin-1-yl]-5-(propan-2-yl)isoquinoline as yellow solid.

### 4.1.5 Synthesis of N-(2-((3S,4R)-3-fluoro-4-methoxypiperidin-1-yl)pyrimidin-4-yl)-5-isopropyl-8-((2R,3S)-2-methyl-3-((methylsulfonyl)methyl)azetidin-1-yl)isoquinolin-3-amine

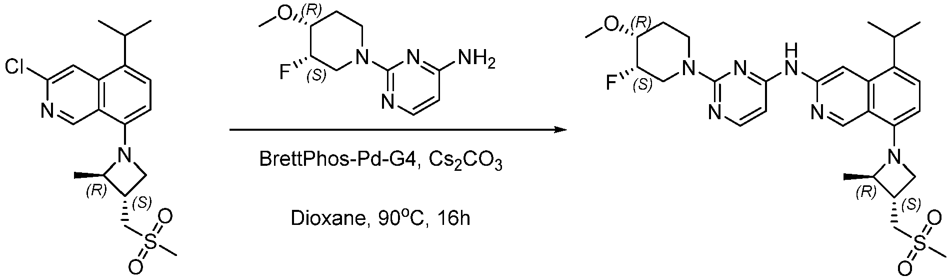

To a solution of 2-((3S,4R)-3-fluoro-4-methoxypiperidin-1-yl)pyrimidin-4-amine (18.50 mg, 0.082 mmol, 1 equiv.), 3-chloro-5-isopropyl-8-((2R,3S)-2-methyl-3-((methylsulfonyl)methyl)azetidin-1-yl)isoquinoline (30 mg, 0.082 mmol, 1 equiv.) and Cs₂CO₃ (53.3 mg, 0.164 mmol, 2 equiv.) in 1,4-Dioxane (0.82 ml) was added BrettPhos Precatalyst (Gen IV) (3.76 mg, 4.09 µmol, 0.05 equiv.) under N₂, the mixture was stirred at 90 °C for 16 h. The mixture was filtered and concentrated in vacuo. The crude mixture was purified by reverse phase chromatography (0 to 60% acetonitrile/water containing 0.1% TFA). Pure fractions were combined and neutralized with saturated sodium bicarbonate solution and then extracted with 10% MeOH/DCM (5 mL x 3). Combined organic phases dried over sodium sulfate, filtered and evaporated to give 17.4 mg of the title compound (38%) as a yellow solid.

XRPD diffractogram of the obtained product demonstrated that the solid was a crystalline material which was denoted as Form A. It was later determined that the obtained product contains XPhos related impurities which show peaks at 2θ of ~8.34 and ~9.54 in the XRPD.

### 4.2 Preparations of Compound (I) Form A

### Method A: Amorphous Slurries

Approximately 20-25 mg of the amorphous Compound (I) (see Example 6) were placed in 2 mL vials with 5 mm stir bars. The respective solvent was added to the vials by mixing with a stirring rate of 300 rpm at RT and the clear solutions/slurries were stirred at RT. In most cases, the solids dissolved completely into clear solutions and were stirred continuously without adding additional solids. Thin or very thick slurries were observed from the clear solutions in 2 min to 1 h. The slurries were sampled on day 0 (as soon as the slurries were observed), day 1, day 4, and day 5. The observations were recorded and are summarized below in Table 4.

**Table 4. Summary of XRPD patterns of solids obtained from amorphous slurry experiments with of Compound (I).**

| NT = Not Tested ; N/A = No Solid to Test | | | | | |
|---|---|---|---|---|---|
| **Solvent** | **Solvent Quantity (µL)** | **XRPD Pattern** | | | |
| | | **Day 0** | **Day 1** | **Day 4** | **Day 5** |
| 2-MeTHF | 150 | Form A | Form A | Form A | NT |
| 1,4-Dioxane | 100 | Form A | Form A | Form A | NT |
| EtOH | 100 | Form A | Form A | Form A | NT |
| ACN:water (8:2 vol.) | 100 | Form A | N/A | Form A | NT |
| MeOAc | 150 | Form A | Form A | Form A | NT |
| MeOH | 100 | Form A | Form A | Form A | NT |
| DMSO | 2 vol | N/A | N/A | N/A | A |

### Method B: Amorphous Vapor Diffusion

Approximately 10-20 mg of the amorphous Compound (I) were weighed into 2 mL vials and the vials were placed in 20 mL scintillation vials containing 2 mL of the respective diffusing solvent. The 20 mL scintillation vials were sealed with caps and parafilm. The observations were made immediately after exposure of the amorphous solid into vapor diffusion and the are summarized in Table 5. In most of the cases, the fluffy amorphous solids were observed to shrink into a dark-yellow thin layer at the bottom of the vial.

**Table 5. Summary of XRPD patterns of solids obtained from amorphous slurry experiments with Compound (I).**

| **Diffusing Solvent** | **Weight of amorphous Compound (I) (mg)** | **XRPD Pattern** | | **Observations** |
|---|---|---|---|---|
| | | **Day 1** | **Day 7** | |
| MeOH | 17.0 | Form A | NT | Shrinking of the fluffy solid into a thin layer of dark-yellow solid in 5 min of vapor diffusion, which turned to a pale-yellow solid after 2 h. |
| MEK | 15.8 | Form A | NT | Shrinking of the fluffy solid into a thin layer of dark-yellow solid in 5 min of vapor diffusion, which turned to a pale- yellow solid after 2 h. |
| | | | | The solid was slightly wet. |
| MeOAc | 18.4 | Form A | NT | Shrinking of the fluffy solid into a thin layer of dark-yellow solid in 5 min of vapor diffusion, which turned to a pale-yellow solid after 2 h. |
| 2-MeTHF | 17.1 | Form A | Form A | Shrinking of the fluffy solid into a thin layer of dark-yellow solid in 30 min of vapor diffusion, which turned to a pale-yellow solid after 2 h. |
| Water | 19.5 | Amorphous | NT | The solid remained same with no change even after 5 days of vapor diffusion. |
| ACN | 17.7 | Form A | NT | Shrinking of the fluffy solid into a thin layer of dark-yellow solid in 10 min of vapor diffusion, which turned to a pale-yellow solid after 2 h. |
| MtBE | 13.6 | Amorphous | NT | The solid remained same with no change even after 5 days of vapor diffusion. |
| 1,4-Dioxane | 9.6 | Form A | Form A | Shrinking of the fluffy solid into a thin layer of dark-yellow solid after overnight vapor diffusion, which turned to a pale-yellow solid after 2 h. |
| | | | | The solid was wet with a very little amount of solvent diffused inside the vial. |
| EtOH | 9.5 | Form A | NT | Shrinking of the fluffy solid into a thin layer of dark-yellow solid in 30 min of vapor diffusion, which turned to a pale-yellow solid after 2 h. |
| | | | | The diffused solid was a dry powder. |
| THF | 10.5 | Form A | NT | Shrinking of the fluffy solid into a thin layer of dark-yellow solid in 5 min of vapor diffusion, which turned to a pale-yellow solid after 2 h. |
| | | | | The solid was wet with a few drops of solvent diffused inside the vial. |

| | | | | |
|---|---|---|---|---|
| NT = Not Tested | | | | |

### Method C: Reverse Addition Antisolvent Crystallization

Approximately 25-35 mg of the amorphous Compound (I) was dissolved in the solvent mostly at RT. Twice the amount of antisolvent was taken in a separate vial, to which the solution was added as one transfer with rapid stirring. For example, if solids dissolved in 0.5 mL of solvent, then the solution was added to 1.0 mL of antisolvent as one transfer with vigorous stirring. Once solids were formed, the slurries were filtered, and the recovered solids were analyzed by XRPD. The results of antisolvent crystallizations in reverse additions are shown in Table 6.

Only in MEK/n-heptane, a medium-thin slurry formed in 15 min after the solution was added to the antisolvent by vigorous stirring. The slurry was filtered and the solid recovered was analyzed by XRPD. A hazy solution formed in tetrahydrofuran (THF)/EtOH 2 h after adding solution to the antisolvent with continued stirring (~600 rpm). The hazy solution turned to a thin slurry, which was filtered under reduced pressure and the solid was analyzed by XRPD. Also, hazy solutions formed in THF/isopropyl acetate (IPAc) and THF/toluene, 3 h after adding solution to antisolvent with continued stirring at RT. For other solvents, the clear solutions were transferred to a chiller block at -5 °C with continued stirring for one week. The clear solutions remained clear, and the vials were transferred to the freezer at -20 °C. A small amount of crystalline solids or precipitate were observed.

**Table 6. Summary of XRPD patterns of solids obtained from reverse addition antisolvent crystallization of Compound (I).**

| **Solvent** | **Antisolvent** | **XRPD Pattern** | **Observations** |
|---|---|---|---|
| MEK | *n*-Heptane | Form A | Thin slurry was syringe filtered to antisolvent. |
| | | | Yellow hazy solution formed initially turned to slurry in 15 min. |
| | EtOH | Form A | Thin slurry was syringe filtered to antisolvent. |
| | | | Yellow clear solution. |
| | | | A small amount of crystalline solids after four weeks in the freezer at -20 °C. |
| 1,4-Dioxane | IPAc | Form A | Clear yellow solution added to antisolvent. |
| | | | Clear yellow solution. |
| | | | A small amount of crystalline solids after four weeks in the freezer at -20 °C. |
| | *n*-PA | Form A | Clear yellow solution added to antisolvent. |
| | | | Very thin slurry. |
| | | | A portion was filtered but very small amount of solid was recovered. |
| | Toluene | Form A | Clear yellow solution added to antisolvent. |
| | | | Clear yellow solution. |
| | | | A small amount of crystalline solids after four weeks in the freezer at -20 °C. |
| THF | IPAc | Form A | Clear solution turned hazy in 3 h, which turned to a medium-thin slurry overnight. |
| | EtOH | Form A | Clear solution turned hazy in 2 h, which turned to a medium-thin slurry in 6 h. |
| | Toluene | Form A | Clear solution turned hazy in 3 h, which turned to a medium-thin slurry overnight. |

### Method D: Direct Addition Antisolvent Crystallization

Approximately 22-35 mg of the amorphous Compound (I) was taken in either 2 mL or 4 mL vials, depending upon the solvent required to dissolve the solid. The solvent was added to the solids and the thin/medium slurries were stirred at 30 °C with stir bars (5 mm for 2 mL vials and 10 mm thin bars for 4 mL vials) for up to 1 h to obtain a clear solution. Twice the volume of solvent was used as antisolvent. For example, if solids dissolved in 0.5 mL solvent, then 1.0 mL of the antisolvent was used for the direct addition. The antisolvent was added in four equal portions dropwise to the vigorously stirring solution over an hour.

In the cases of MEK, 1,4-dioxane, and THF, thin slurries formed on adding the solvent to the solids, which remained even after stirring for 1 h at 30 °C. However, the thin slurries were dissolved into clear solution by heating the vials at 40 °C for less than 5 min. The vials were then transferred to RT and the antisolvent was added. In the case of 2-methyltetrahydrofuran (2-MeTHF): dimethyl sulfoxide (DMSO) (9:1 vol.), the solids dissolved in 7 vol of the solvent. Thehazy solutions/very thin slurries were continuously stirred at RT for 2 days and medium to thick slurries were observed in most of the cases. The summary of the experiments is given Table 7.

**Table 7. Summary of XRPD patterns of solids obtained from direct addition antisolvent crystallization of Compound (I).**

| **Solvent** | **Antisolvent** | **XRPD Pattern** | **Observations** |
|---|---|---|---|
| MEK | *n*-Heptane | Form A | A hazy solution on first addition of antisolvent turned to thin slurry on third addition. |
| | | | The slurry was filtered after fourth addition. |
| 1,4-Dioxane | IPAc | Form A | Solution turned to slightly hazy on first addition, which turned to fully hazy after fourth addition. |
| | | | Medium-thick slurry was observed after 2 days of stirring at RT. |
| | *n*-PA | Form A | Solution turned to slightly hazy on fourth addition. |
| | | | Thin slurry was observed after 2 days of stirring at RT. |
| | Toluene | Form A | Solution remained clear even after fourth addition. |
| | | | Hazy solution after 2 days of stirring at RT. |
| | | | Thin slurry after 4 days was filtered. |
| THF | IPAc | Form A | Slightly hazy on first addition, which turned to a thin slurry on second addition. |
| | | | The slurry was filtered after second addition. |
| | EtOH | Form A | Slightly hazy on first addition, which turned to a thin slurry on second addition. |
| | | | The slurry was filtered after second addition. |
| | Toluene | Form A | Slightly hazy on first addition, which turned to a thin slurry on second addition. |
| | | | The slurry was filtered after second addition. |
| 2-MeTHF : DMSO (9:1 vol) | IPAc | Form A | Solution turned to lightly hazy on second addition, which turned to fully hazy after fourth addition. |
| | | | Medium-thick slurry was observed after 2 days of stirring at RT. |
| | EtOH | Form A | Solution turned to a thin slurry after second addition. |
| | | | Thin slurry was filtered after second addition. |
| | Toluene | Form A | Clear solution remained after fourth addition. |
| | | | Medium-thick slurry was observed after 2 days of stirring at RT. |

### Method E: Fast Cooling Crystallizations

Approximately 25-35 mg of the amorphous Compound (I) was weighed into 2 mL vial. The solvents and 5 mm stir bars were added to vial and stirred at 50 °C at a stirring rate of 450 rpm. The thin slurry/hazy solutions were heated to 60 °C to obtain clear solutions if required. The clear solutions were transferred to an ice-water bath near 0 °C without mixing. Care was taken to ensure no visible crust was present prior to cooling of the samples. In general, no slurries or solids were observed in fast-cooling crystallization upon transferring solutions to the ice-water bath. However, in some cases hazy solutions were observed after allowing the solutions to stir at RT on stir plate inside the ice-water bath for up to 1 h and solids obtained after being transferred to the freezer at -20 °C. The summary of the fast-cooling crystallization is given in Table 8.

**Table 8. Summary of XRPD patterns of solids obtained from fast-cooling crystallization of Compound (I).**

| **Solvent** | **XRPD Pattern** | **Observations** |
|---|---|---|
| 1,4-Dioxane :EtOH (8:2 vol.) | Form A | Thin slurry formed at 50 °C, which turned to clear solution by heating it to 60 °C. |
| | | Thick slurry formed after overnight in freezer (-20 °C) |
| MEK | Form A | Clear solution at 50 °C. |
| | | Hazy solution formed in 5 min in ice-water bath. Trace of solids. |
| | | A few yellow solids in the vial after five weeks in the freezer at -20 °C. |
| MeOAc | Form A | Clear solution in 29 vol. at 60 °C. |
| | | Clear solution remained in freezer (-20 °C). |
| | | Thin slurry was observed after 12 days in the freezer and was filtered. |
| 2-MeTHF | Form A | Solid remained a thin slurry even after heating to 60 °C. |
| | | Medium-thin slurry was observed after 9 days in the freezer and was filtered. |

### Method F: Slow Cooling Crystallizations

Approximately 25-35 mg of the amorphous Compound (I) was weighed into 2 mL vials. The solvents and 5 mm stir bars were added to vial and stirred at 50 °C at a stirring rate of 450 rpm. In all the cases, thin slurries were formed at ~50 °C due to the concentration of the solids in the solvents. Hence, the vials were heated up to 67 °C to obtain clear solutions. Care was taken to ensure no visible crust was present prior to cooling of the samples. The clear solutions at 67 °C were then cooled to RT with the cooling rate of 5 °C/h. This was achieved by reducing the temperature of hotplate by 2.5 °C every 30 min. The summary of the slow-cooling crystallization is given in Table 9.

**Table 9. Summary of XRPD patterns of solids obtained from slow-cooling crystallization of Compound (I).**

| **Solvent** | **XRPD Pattern** | **Observations** |
|---|---|---|
| 1,4-Dioxane :EtOH (8:2 vol.) | Form A | Clear solution at RT, which turned to a thick pale yellow slurry after 2 days of stirring at RT. |
| 2-MeTHF | Form A | Clear solution at RT, which turned to a medium-thick pale-yellow slurry after 2 days of stirring at RT. |
| THF | Form A | Clear solution at RT, which turned to a thick pale yellow slurry after 2 days of stirring at RT. |

### Method G: Stagnant Cooling Crystallizations

Approximately 25-35 mg of the amorphous Compound (I) was weighed into 2 mL vials. The solvents and 5 mm stir bars were added to the vial and stirred at 50 °C at a stirring rate of 450 rpm. The thin slurry/hazy solutions were heated to 60 °C to obtain clear solutions, if required. The clear solutions were transferred to a freezer at -20 °C and crystallization process was observed periodically. Care was taken to ensure no visible crust was present prior to cooling of the samples. The summary of the experiments is given in Table 10.

**Table 10. Summary of XRPD patterns of solids obtained from stagnant cooling crystallization of Compound (I).**

| **Solvent** | **XRPD Pattern** | **Observations** |
|---|---|---|
| 1,4-Dioxane :EtOH (8:2 vol.) | Form A | Thin slurry formed at 50 °C, which turned to clear solution by heating to 60 °C. |
| | | Thick slurry formed after overnight in freezer (-20 °C). |
| 2-MeTHF | Form A | Solid remained a thin slurry even after heating to 60 °C. |
| | | Syringe filtered, and the solution was transferred to the freezer. Trace of solids. A thin slurry, observed after 11 days, was filtered. |
| MeOAc | Form A | Thin slurry formed at 50 °C, which turned to clear solution by heating to 60 °C. |
| | | Thick slurry formed after overnight in freezer (-20 °C). |
| MEK | Form A | Thin slurry formed at 50 °C, which turned to clear solution by heating to 60 °C. |
| | | Remained clear solution in the freezer (-20 °C). |
| | | A small amount of yellow solid was observed in the vial after 5 weeks in the freezer at -20 °C. |

### Method H: Slow Evaporation Crystallizations

Approximately 25-35 mg of the amorphous Compound (I) was weighed in 2 mL or 4 mL vials depending on the amount of solvent required to completely dissolve the solids into clear solutions. The solvents were added to the vials by mixing with 5 mm (for 2 mL vials) or 10 mm (for 4 mL vials) stir bars on the stir plate at RT at a stirring rate of 300 rpm. Once the solids were dissolved completely into clear solutions, the vials were capped or sealed, and the caps were pinned with high gauge syringe needles to allow solvents to slowly evaporate from the vials. The solutions continued to stir during the slow evaporation. The summary of the experiments is given in Table 11.

**Table 11. Summary of XRPD patterns of solids obtained from slow evaporation crystallization of Compound (I).**

| **Solvent** | **XRPD Pattern** | **Observations** |
|---|---|---|
| DCM | Form A | Very thick slurry formed after 4 days |
| THF | Form A | A thick pale-yellow slurry after 12 days of stirring at RT |

### Method I: Flash Evaporation Crystallizations

Approximately 25-35 mg of the amorphous Compound (I) was weighed in 4 mL vials and the solvents were added by mixing with 10 mm stir bars. The microscope slides were placed on preheated hotplate at ~115 °C. The clear solutions were added dropwise using glass pipette on the hot microscope slides. The solutions were evaporated instantly while dropping on the slides and overall, the solutions turned to fluffy solids in less than 2 min. The solids were recovered by scrapping the slides by spatula and used for analysis. The summary of the flash evaporation experiments is given in Table 12.

**Table 12. Summary of XRPD patterns of solids obtained from flash evaporation crystallization of Compound (I).**

| **Solvent** | **XRPD Pattern** | **Observations** |
|---|---|---|
| Acetone | Form A | Dissolved completely into clear solution at RT. |
| | | Fluffy solid. |
| DCM | Amorphous | Dissolved completely into clear solution at RT. |
| | | Fluffy solid. |
| MEK | Form A | Dissolved completely into clear solution at RT. |
| | | Fluffy solid. |
| MeOAc | Amorphous | Very thin slurry was syringe filtered. |
| | | Fluffy solid. |
| THF | Amorphous | Dissolved completely into clear solution at RT. |
| | | Fluffy solid. |
| 2-MeTHF | Form A | Very thin slurry was syringe filtered. |
| | | Fluffy solid. |

### Method J: Using Crystallographic Template

Slow evaporation in the presence of crystallographic template was carried out with clear solutions obtained in the presence of the templates. A ground mixture of various minerals (fluorite, garnet, pyrite, apophyllite, dolomite, corundum, tourmaline, topaz, celestite, staurolite, diopside, and amazonite) were used as crystallographic templates for this purpose. The solutions with the templates were allowed to evaporate with the sealed caps, pinned with high gauge syringe needles to allow solvents to evaporate from the vials by mixing the solutions. The summary of the slow evaporation with and crystallography template are given in Table 13.

**Table 13. Summary of XRPD patterns of solids obtained from slow evaporation of Compound (I) in the presence of a crystallographic template.**

| **Solvent** | **XRPD Pattern** | **Observations** |
|---|---|---|
| MeOAc | Form A | Thin slurry after two weeks |
| DCM | Form A | Very thick slurry formed after 4 days |
| THF | Form A | Thin slurry after two weeks |

### 4.3 Characterization of Compound (I) Form A

The obtained Compound (I) Form A was characterized by XRPD using the High Resolution Scan Method (see Figure 10 and Table 14) and TGA-DSC (Figure 11).

The combined DSC and TGA thermogram showed a total mass loss of 0.7 wt.% and endotherm onsets at 170.53 °C and 196.81 °C (Figure 11).

**Table 14. Peak list for XRPD pattern of Compound (I) Freebase Form A**

| **2θ (deg)** | **d-Spacing (ang.)** | **Relative Intensity (%)** |
|---|---|---|
| 6.72 | 13.13 | 19 |
| 7.98 | 11.07 | 35 |
| 9.87 | 8.96 | 100 |
| 10.24 | 8.63 | 5 |
| 11.77 | 7.51 | 29 |
| 12.08 | 7.32 | 83 |
| 12.53 | 7.06 | 14 |
| 13.82 | 6.40 | 16 |
| 14.45 | 6.12 | 94 |
| 14.84 | 5.96 | 5 |
| 15.72 | 5.63 | 13 |
| 15.94 | 5.56 | 77 |
| 17.72 | 5.00 | 16 |
| 18.07 | 4.91 | 10 |
| 18.66 | 4.75 | 22 |
| 19.37 | 4.58 | 32 |
| 19.65 | 4.51 | 47 |
| 20.12 | 4.41 | 67 |
| 20.51 | 4.33 | 24 |
| 20.69 | 4.29 | 57 |
| 20.96 | 4.24 | 15 |
| 21.80 | 4.07 | 8 |
| 21.99 | 4.04 | 21 |
| 22.75 | 3.91 | 24 |
| 23.04 | 3.86 | 7 |
| 23.60 | 3.77 | 10 |
| 23.81 | 3.73 | 22 |
| 24.24 | 3.67 | 9 |
| 29.28 | 3.05 | 8 |
| 29.78 | 3.00 | 7 |
| 30.93 | 2.89 | 7 |

### Example 5: Preparations and Characterization of Crystalline Form B of Compound (I) Free Base

### 5.1 Preparation

Form B of Compound (I) (351.2 mg) was made via reverse antisolvent addition with DMAc/water as described above for Form A. The thick light-yellow slurry was filtered and washed with 1 × 2.0 vol. of water and left on the filter paper for 5 min with active suction from the aspirator. The sample was then placed in the oven at 50 °C under active vacuum for 15 min and then left on the benchtop overnight to dry. After drying, The solid obtained was further characterized by XRPD using the High-Resolution Scan Method (see Figure 12 and Table 15), TGA-DSC (Figures 15 and 16), and DVS.

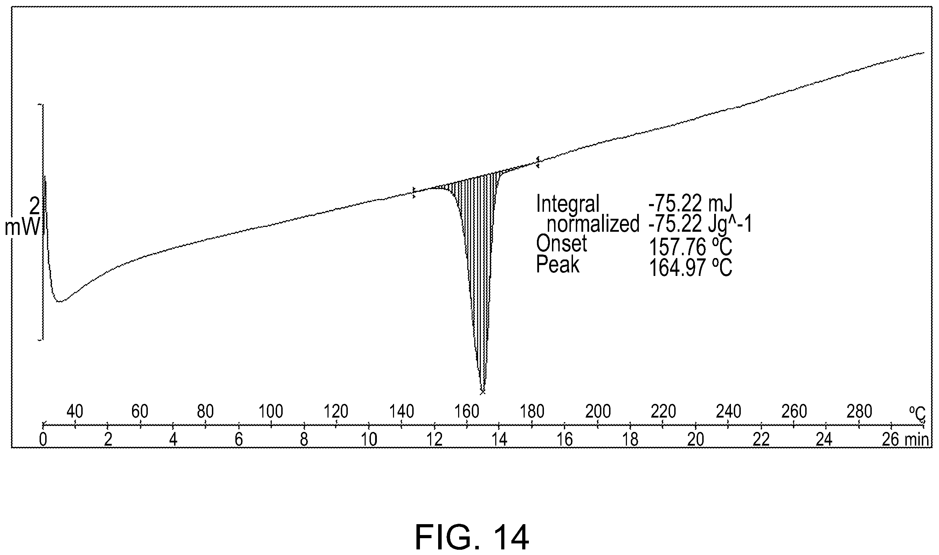

The combined DSC and TGA thermogram showed a total mass loss of 0.1 wt.% and an endotherm onset at 158.7 °C (Figure 13). The DSC alone showed an endotherm onset at 157.8 °C (Figure 14).

**Table 15. Peak list for XRPD pattern of Compound (I) Freebase Form B**

| **2θ (deg)** | **d-Spacing (ang.)** | **Relative Intensity (%)** |
|---|---|---|
| 5.08 | 17.39 | 78 |
| 10.13 | 8.72 | 10 |
| 12.17 | 7.27 | 55 |
| 13.48 | 6.56 | 100 |
| 15.20 | 5.82 | 12 |
| 16.34 | 5.42 | 19 |
| 16.63 | 5.33 | 52 |
| 17.69 | 5.01 | 9 |
| 17.89 | 4.95 | 11 |
| 19.50 | 4.55 | 19 |
| 20.09 | 4.42 | 43 |
| 20.35 | 4.36 | 36 |
| 20.65 | 4.30 | 11 |
| 20.90 | 4.25 | 11 |
| 21.40 | 4.15 | 31 |
| 22.70 | 3.91 | 14 |
| 23.78 | 3.74 | 10 |
| 25.17 | 3.54 | 16 |
| 25.41 | 3.50 | 5 |
| 26.27 | 3.39 | 12 |
| 27.04 | 3.29 | 7 |
| 31.01 | 2.88 | 8 |

### 5.2 DVS of Compound (I) Freebase Form B

DVS was completed and showed a mass change of 2.1 wt. % between 2 and 95% relative humidity at 25 °C.

### Example 6: Preparations and Characterization of Amorphous Compound (I)

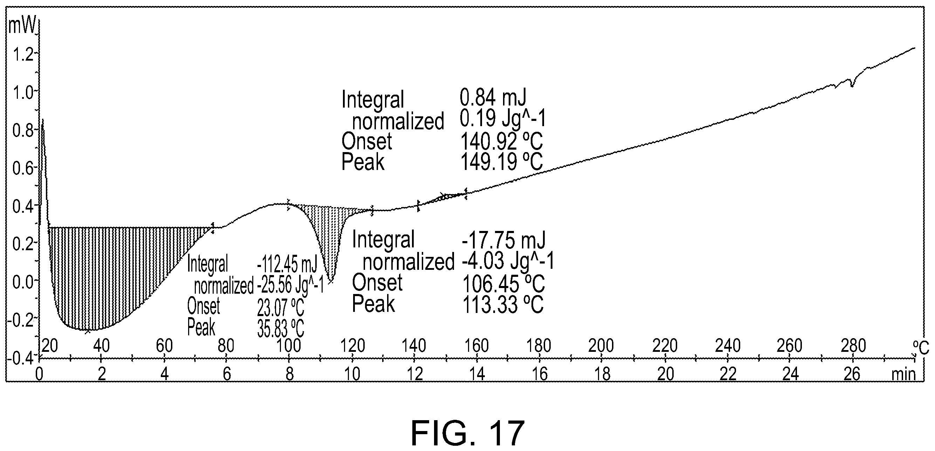

Approximately 263 mg of the Compound (I) was weighed into a 20 mL scintillation vial and 10 mm stir bar was added. To the vial was added 8 mL of ACN:water (8:2 vol.) by mixing at RT and a medium-thin slurry was formed. The slurry was stirred at ~55 °C for 10 min and it turned to a thin slurry. Additional solvent (2 mL) was added and the thin slurry continued to stir for another 10 min at ~55 °C. A very thin slurry remaining after 10 min was syringe filtered using 45 µm filter into another clean 20 mL scintillation vial. The clear yellow solution was freeze-dried by placing the vial in liquid nitrogen for 2-3 min. The vial with fully frozen solid was lyophilized overnight. The solid recovered after overnight lyophilization was fluffy and amorphous in nature. The solid obtained was further characterized by XRPD using the Regular Scan Method (see Figure 15) and TGA-DSC (Figures 16 and 17).

The combined DSC and TGA thermogram showed onset endotherms at 30.7 °C and 108.7 °C (Figure 16). The DSC alone showed onset endotherms at 23.1 °C and 106.5 °C (Figure 17).

## Claims

1. A salt of Compound (I), represented by the following structural formula: 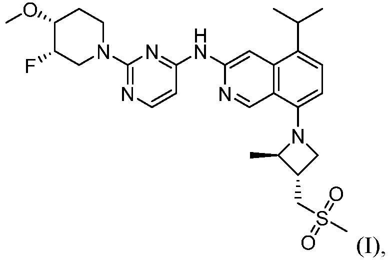 wherein the salt is a succinate salt and the molar ratio between compound (I) and succinic acid is 1:0.5, or wherein the salt is a glutarate salt and the molar ratio between compound (I) and glutaric acid is 1:0.5, or wherein the salt is a fumarate salt and the molar ratio between compound (I) and fumaric acid is 1:1.

2. The succinate salt of claim 1, wherein the succinate salt is crystalline.

3. The succinate salt of claim 2, wherein said crystalline succinate salt is crystalline Form C, **characterized by**:
(i) an X-ray powder diffraction pattern which comprises at least three or four peaks chosen from 4.5°, 8.9°, 9.3°, 15.3°, and 17.8° ± 0.2 in 2θ;
(ii) an X-ray powder diffraction pattern which comprises peaks at 4.5°, 8.9°, 9.3°, 15.3°, and 17.8° ± 0.2 in 2θ
(iii) an X-ray powder diffraction pattern which comprises at least three, four, five, six, seven, eight, nine, or ten peaks chosen from 4.5°, 8.9°, 9.3°, 13.0°, 15.3°, 16.8°, 17.8°, 18.1°, 18.5°, and 22.3° ± 0.2 in 2θ;
(iv) an X-ray powder diffraction pattern which comprises peaks at 4.5°, 6.7°, 8.9°, 9.3°, 11.1°, 12.3°, 13.0°, 14.4°, 15.3°, 16.3°, 16.8°, 17.8°, 18.1°, 18.5°, 20.5°, 22.3°, and 26.0° ± 0.2 in 2θ; or
(v) an X-ray powder diffraction pattern substantially similar to Figure 1; and/or
wherein said crystalline succinate salt is Form C, **characterized by** a differential scanning calorimeter with an onset temperature of 175 ± 2°C; and/or
wherein said crystalline succinate salt is Form C, **characterized by** a thermogravimetric analysis (TGA) substantially similar to Figure 2; and/or wherein at least 90% by weight of the succinate salt is crystalline Form C.

4. The succinate salt of any one of claims 1-3, obtained by a process comprising:
combining Compound (I) and succinic acid in ethyl acetate;
collecting said succinate salt of Compound (I);
adding 2-propanol to said succinate salt of Compound (I); and
recollecting said succinate salt of Compound (I).

5. The glutarate salt of claim 1, wherein said glutarate salt is crystalline.

6. The glutarate salt of claim 5, wherein said crystalline glutarate salt is Form D, **characterized by**:
(i) an X-ray powder diffraction pattern which comprises at least three or four peaks chosen from 8.8°, 14.8°, 16.1°. 18.3°, and 18.7° ± 0.2 in 2θ;
(ii) an X-ray powder diffraction pattern which comprises peaks at 8.8°, 14.8°, 16.1°. 18.3°, and 18.7° ± 0.2 in 2θ;
(iii) an X-ray powder diffraction pattern which comprises at least three, four, five, six, or seven peaks chosen from 7.4°, 8.8°, 12.3°, 14.8°, 16.1°, 18.3°, and 18.7° ± 0.2 in 2θ;
(iv) an X-ray powder diffraction pattern which comprises peaks at 6.6°, 7.4°, 8.8°, 12.3°, 12.9°, 14.8°, 16.1°, 18.3°, 18.7°, 20.0°, and 22.2° ± 0.2 in 2θ; or
(v) an X-ray powder diffraction pattern substantially the same as Figure 4; and/or
wherein said crystalline glutarate salt is Form D, **characterized by** a differential scanning calorimeter with an onset temperature of 142 ± 2°C; and/or
wherein said crystalline glutarate salt is Form D, **characterized by** a thermogravimetric analysis (TGA) substantially similar to Figure 5; and/or wherein at least 90% by weight of the glutarate salt is crystalline Form D.

7. The glutarate salt of any one of claims 1, 5, and 6, obtained by a process comprising:
combining Compound (I) and glutaric acid in ethyl acetate; and
collecting said glutarate salt of Compound (I).

8. The fumarate salt of claim 1, wherein said fumarate salt is crystalline.

9. The fumarate salt of claim 8, wherein said crystalline fumarate salt is Form E, **characterized by**:
(i) an X-ray powder diffraction pattern which comprises at least three, four, or five peaks chosen from 6.3°, 8.5°, 9.0°, 14.5°, 15.7°, and 18.0° ± 0.2 in 2θ;
(ii) an X-ray powder diffraction pattern which comprises peaks at 6.3°, 8.5°, 9.0°, 14.5°, 15.7°, and 18.0° ± 0.2 in 2θ;
(iii) an X-ray powder diffraction pattern which comprises at least three, four, five, six, seven, eight, nine, or ten peaks chosen from 6.3°, 8.5°, 9.0°, 12.1°, 14.5°, 15.7°, 18.0°, 19.7°, 20.1°, and 21.9° ± 0.2 in 2θ;
(iv) an X-ray powder diffraction pattern which comprises peaks at 6.3°, 8.5°, 9.0°, 12.1°, 14.5°, 15.1°, 15.2°, 15.4°, 15.7°, 18.0°, 18.2°, 18.9°, 19.3°, 19.7°, 20.1°, 20.6°, 20.7°, 21.3°, and 21.9° ± 0.2 in 2θ; or
(v) an X-ray powder diffraction pattern substantially similar to Figure 7; and/or
wherein said crystalline fumarate salt is Form E, **characterized by** a differential scanning calorimeter with an onset temperature of 164 ± 3°C; and/or
wherein said crystalline fumarate salt is Form E, **characterized by** a thermogravimetric analysis (TGA) substantially similar to Figure 8; and/or
wherein at least 90% by weight of the fumarate salt is crystalline Form E.

10. The fumarate salt of any one of claims 1, 8, and 9, obtained by a process comprising:
combining Compound (I) and fumaric acid in ethyl acetate;
removing said ethyl acetate to give said fumarate salt of Compound (I);
adding trifluoroethanol to said fumarate salt of Compound (I);
removing said trifluoroethanol to give said fumarate salt of Compound (I); and
adding ethyl acetate to said fumarate salt of Compound (I).

11. A freebase crystalline Form B of compound (I), represented by the following structural formula: 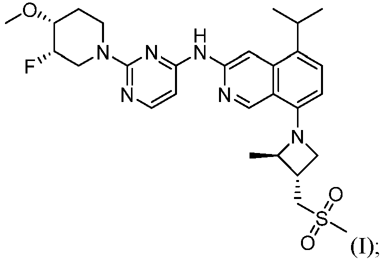
wherein said Form B is **characterized by** an X-ray powder diffraction pattern which comprises at least three, four, or five peaks chosen from 5.1°, 12.2°, 13.5°, 16.6°, and 20.1° ± 0.2 in 2θ, wherein said Form B is optionally further **characterized by**:
(i) an X-ray powder diffraction pattern which comprises at least three, four, five, six, seven, eight, nine, ten, or eleven peaks chosen from 5.1°, 12.2°, 13.5°, 16.3°, 16.6°, 19.5°, 20.1°, 20.4°, 21.4°, 22.7°, and 25.2° ± 0.2 in 2θ;
(ii) an X-ray powder diffraction pattern which comprises peaks at 5.1°, 12.2°, 13.5°, 15.2°, 16.3°, 16.6°, 17.9°, 19.5°, 20.1°, 20.4°, 20.7°, 20.9°, 21.4°, 22.7°, 25.2°, and 26.3° ± 0.2 in 2θ; or
(iii) an X-ray powder diffraction pattern substantially similar to Figure 12; and/or
wherein said Form B is optionally further **characterized by** a differential scanning calorimeter with an onset temperature of 158 ± 2°C; and/or
wherein said Form B is optionally further **characterized by** a thermogravimetric analysis (TGA) substantially similar to Figure 13; and/or
optionally wherein at least 90% by weight of the freebase is crystalline Form B.

12. The freebase crystalline Form B of claim 11, obtained by a process comprising:
adding Compound (I) to dimethylacetamide to form a mixture; and
adding said mixture to water to obtain Compound (I) freebase crystalline Form B.

13. An amorphous form of Compound (I) freebase represented by the following structural formula: 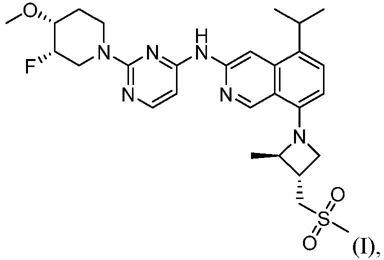 wherein said amorphous form is **characterized by** a differential scanning calorimeter with an onset temperature of 157 ± 2°C.

14. The amorphous form of claim 13, obtained by a process comprising:
adding an acetonitrile : water mixture to Compound (I) to form a solution;
filtering said solution;
freezing said solution; and
lyophilizing said frozen solution to obtain said amorphous Compound (I).

15. A pharmaceutical composition comprising said salt of any one of claims 1-10, or said freebase of any one of claims 11-14, and a pharmaceutically acceptable carrier or a diluent.

16. The salt of any one of claims 1-10, the freebase of any one of claims 11-14, or the pharmaceutical composition of claim 15, for use in a method of treating cancer in a subject, optionally wherein:
(i) said cancer is non-small cell lung cancer;
(ii) said subject has at least one mutation in the EGFR gene that results in the expression of an EGFR enzyme with amino acid modification selected from the group consisting of L858R, T790M, C797S, and combinations thereof, optionally wherein said EGFR gene has amino acid modifications of L858R, T790M, and C797S; and/or
(iii) the method further comprises administering said subject an effective amount of afatinib, osimertinib, erlotinib, or gefitinib.

17. The salt of any one of claims 1-10, the freebase of any one of claims 11-14, or the pharmaceutical composition of claim 15, for use in a method of inhibiting epidermal growth factor receptor (EGFR) in a subject in need thereof.

## Patentansprüche

1. Ein Salz der Verbindung (I), dargestellt durch die folgende Strukturformel: 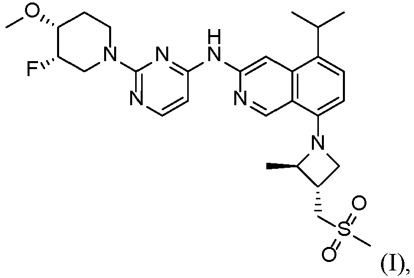 wobei das Salz ein Succinatsalz ist und das Molverhältnis zwischen Verbindung (I) und Bernsteinsäure 1:0,5 beträgt, oder wobei das Salz ein Glutaratsalz ist und das Molverhältnis zwischen Verbindung (I) und Glutarsäure 1:0,5 beträgt, oder wobei das Salz ein Fumaratsalz ist und das Molverhältnis zwischen Verbindung (I) und Fumarsäure 1:1 beträgt.

2. Succinatsalz nach Anspruch 1, wobei das Succinatsalz kristallin ist.

3. Succinatsalz nach Anspruch 2, wobei das genannte kristalline Succinatsalz kristalline Form C ist, **gekennzeichnet durch**:
(i) ein Röntgenpulverbeugungsmuster, das mindestens drei oder vier Peaks ausgewählt aus 4,5°, 8,9°, 9,3°, 15,3° und 17,8° ± 0,2, in 2θ umfasst;
(ii) ein Röntgenpulverbeugungsmuster, das Peaks bei 4,5°, 8,9°, 9,3°, 15,3° und 17,8° ± 0,2, in 2θ umfasst;
(iii) ein Röntgenpulverbeugungsmuster, das mindestens drei, vier, fünf, sechs, sieben, acht, neun oder zehn Peaks, ausgewählt aus 4,5°, 8,9°, 9,3°, 13,0°, 15,3°, 16,8°, 17,8°, 18,1°, 18,5° und 22,3° ± 0,2, in 2θ umfasst;
(iv) ein Röntgenpulverbeugungsmuster, das Peaks bei 4,5°, 6,7°, 8,9°, 9,3°, 11,1°, 12,3°, 13,0°, 14,4°, 15,3°, 16,3°, 16,8°, 17,8°, 18,1°, 18,5°, 20,5°, 22,3° und 26,0° ± 0,2, in 2θ umfasst; oder
(v) ein Röntgenpulverbeugungsmuster, das im Wesentlichen Figur 1 ähnelt; und/oder
wobei das genannte kristalline Succinatsalz Form C ist, **gekennzeichnet durch** ein Differentialscanningkalorimeter mit einer Onset-Temperatur von 175 ± 2°C; und/oder
wobei das genannte kristalline Succinatsalz Form C ist, **gekennzeichnet durch** eine thermogravimetrische Analyse (TGA), die im Wesentlichen Figur 2 ähnelt; und/oder wobei mindestens 90 Gewichts-% des Succinatsalzes kristalline Form C ist.

4. Succinatsalz nach einem der Ansprüche 1-3, erhalten durch einen Prozess, der Folgendes umfasst:
Kombinieren von Verbindung (I) und Bernsteinsäure in Ethylacetat;
Sammeln des genannten Succinatsalzes der Verbindung (I);
Zugeben von 2-Propanol zu dem genannten Succinatsalz von Verbindung (I); und
erneutes Sammeln des genannten Succinatsalzes von Verbindung (I).

5. Glutaratsalz nach Anspruch 1, wobei das genannte Glutaratsalz kristallin ist.

6. Glutaratsalz nach Anspruch 5, wobei das genannte kristalline Glutaratsalz Form D ist, **gekennzeichnet durch**:
(i) ein Röntgenpulverbeugungsmuster, das mindestens drei oder vier Peaks, ausgewählt aus 8,8°, 14,8°, 16,1°, 18,3° und 18,7° ± 0,2, in 2θ umfasst;
(ii) ein Röntgenpulverbeugungsmuster, das Peaks bei 8,8°, 14,8°, 16,1°, 18,3° und 18,7° ± 0,2, in 2θ umfasst;
(iii) ein Röntgenpulverbeugungsmuster, das mindestens drei, vier, fünf, sechs, oder sieben Peaks, ausgewählt aus 7,4°, 8,8°, 12,3°, 14,8°, 16,1°, 18,3° und 18,7° ± 0,2, in 2θ umfasst;
(iv) ein Röntgenpulverbeugungsmuster, das Peaks bei 6,6°, 7,4°, 8,8°, 12,3°, 12,9°, 14,8°, 16,1°, 18,3°, 18,7°, 20,0° und 22,2° ± 0,2, in 2θ umfasst; oder
(v) ein Röntgenpulverbeugungsmuster, das im Wesentlichen das Gleiche wie Figur 4 ist; und/oder
wobei das genannte kristalline Glutaratsalz Form D ist, **gekennzeichnet durch** ein Differentialscanningkalorimeter mit einer Onset-Temperatur von 142 ± 2°C; und/oder
wobei das genannte kristalline Glutaratsalz Form D ist, **gekennzeichnet durch** eine thermogravimetrische Analyse (TGA), die im Wesentlichen Figur 5 ähnelt; und/oder wobei mindestens 90 Gewichts-% des Glutaratsalzes kristalline Form D ist.

7. Glutaratsalz nach einem der Ansprüche 1, 5 und 6, erhalten durch einen Prozess, der Folgendes umfasst:
Kombinieren von Verbindung (I) und Glutarsäure in Ethylacetat; und
Sammeln des genannten Glutaratsalzes von Verbindung (I).

8. Fumaratsalz nach Anspruch 1, wobei das genannte Fumaratsalz kristallin ist.

9. Fumaratsalz nach Anspruch 8, wobei das genannte kristalline Fumaratsalz Form E ist, **gekennzeichnet durch**:
(i) ein Röntgenpulverbeugungsmuster, das mindestens drei, vier oder fünf Peaks, ausgewählt aus 6,3°, 8,5°, 9,0°, 14,5°, 15,7° und 18,0° ± 0,2, in 2θ umfasst;
(ii) ein Röntgenpulverbeugungsmuster, das Peaks bei 6,3°, 8,5°, 9,0°, 14,5°, 15,7° und 18,0° ± 0,2, in 2θ umfasst;
(iii) Röntgenpulverbeugungsmuster, das mindestens drei, vier, fünf, sechs, sieben, acht, neun oder zehn Peaks, ausgewählt aus 6,3°, 8,5°, 9,0°, 12,1°, 14,5°, 15,7°, 18,0°, 19,7°, 20,1° und 21,9° ± 0,2, in 2θ umfasst;
(iv) ein Röntgenpulverbeugungsmuster, das Peaks bei 6,3°, 8,5°, 9,0°, 12,1°, 14,5°, 15,1°, 15,2°, 15,4°, 15,7°, 18,0°, 18,2°, 18,9°, 19,3°, 19,7°, 20,1°, 20,6°, 20,7°, 21,3° und 21,9° ± 0,2, in 2θ umfasst; oder
(v) ein Röntgenpulverbeugungsmuster, das im Wesentlichen Figur 7 ähnelt; und/oder
wobei das genannte kristalline Fumaratsalz Form E ist, **gekennzeichnet durch** ein Differentialscanningkalorimeter mit einer Onset-Temperatur von 164 ± 3°C; und/oder
wobei das genannte kristalline Fumaratsalz Form E ist, **gekennzeichnet durch** eine thermogravimetrische Analyse (TGA), die im Wesentlichen Figur 8 ähnelt; und/oder wobei mindestens 90 Gewichts-% des Fumaratsalzes kristalline Form E ist.

10. Fumaratsalz nach einem der Ansprüche 1, 8 und 9, erhalten durch einen Prozess, der Folgendes umfasst:
Kombinieren von Verbindung (I) und Fumarsäure in Ethylacetat;
Entfernen des genannten Ethylacetats, um das genannte Fumaratsalz von Verbindung (I) zu ergeben;
Zugeben von Trifluorethanol zu dem genannten Fumaratsalz von Verbindung (I);
Entfernen des genannten Trifluorethanols, um das genannte Fumaratsalz von Verbindung (I) zu ergeben; und
Zugeben von Ethylacetat zu dem genannten Fumaratsalz von Verbindung (I).

11. Eine kristalline Freie-Base-Form B von Verbindung (I), dargestellt durch die folgende Strukturformel: 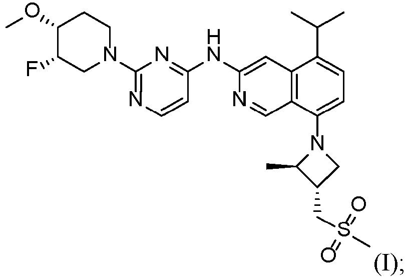
wobei die genannte Form B durch ein Röntgenpulverbeugungsmuster gekennzeichnet ist, das mindestens drei, vier oder fünf Peaks, ausgewählt aus 5,1°, 12,2°, 13,5°, 16,6° und 20,1° ± 0,2, in 2θ umfasst, wobei die genannte Form B optional ferner durch Folgendes gekennzeichnet ist:
(i) ein Röntgenpulverbeugungsmuster, das mindestens drei, vier, fünf, sechs, sieben, acht, neun, zehn oder elf Peaks, ausgewählt aus 5,1°, 12,2°, 13,5°, 16,3°, 16,6°, 19,5°, 20,1°, 20,4°, 21,4°, 22,7° und 25,2° ± 0,2, in 2θ umfasst;
(ii) ein Röntgenpulverbeugungsmuster, das Peaks bei 5,1°, 12,2°, 13,5°, 15,2°, 16,3°, 16,6°, 17,9°, 19,5°, 20, 1°, 20,4°, 20,7°, 20,9°, 21,4°, 22,7°, 25,2° und 26,3° ± 0,2, in 2θ umfasst; oder
(iii) ein Röntgenpulverbeugungsmuster, das im Wesentlichen Figur 12 ähnelt; und/oder
wobei die genannte Form B optional ferner durch ein Differentialscanningkalorimeter mit einer Onset-Temperatur von 158 ± 2°C gekennzeichnet ist; und/oder
wobei die genannte Form B optional ferner durch eine thermogravimetrische Analyse (TGA) gekennzeichnet ist, die im Wesentlichen Figur 13 ähnelt; und/oder
wobei optional mindestens 90 Gewichts-% der freien Base kristalline Form B ist.

12. Die kristalline Freie-Base-Form B nach Anspruch 11, erhalten durch einen Prozess, der Folgendes umfasst:
Zugeben von Verbindung (I) zu Dimethylacetamid, um ein Gemisch zu bilden; und
Zugeben des genannten Gemischs zu Wasser, um die kristalline Freie-Basen-Form B von Verbindung (I) zu erhalten.

13. Eine amorphe Form der freien Base von Verbindung (I), dargestellt durch die folgende Strukturformel: 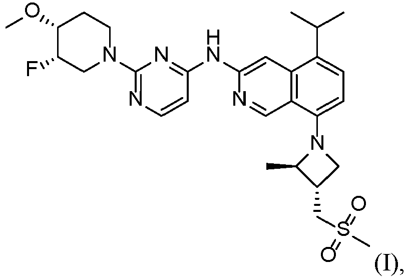 wobei die genannte amorphe Form durch ein Differentialscanningkalorimeter mit einer Onset-Temperatur von 157 ± 2°C gekennzeichnet ist.

14. Amorphe Form nach Anspruch 13, die durch einen Prozess erhalten wird, der Folgendes umfasst:
Zugeben eines Acetonitril:Wasser-Gemischs zu Verbindung (I), um eine Lösung zu bilden;
Filtrieren der genannten Lösung;
Einfrieren der genannten Lösung; und
Lyophilisieren der genannten gefrorenen Lösung, um die genannte amorphe Verbindung (I) zu erhalten.

15. Eine pharmazeutische Zusammensetzung, die das genannte Salz nach einem der Ansprüche 1-10 oder die genannte freie Base nach einem der Ansprüche 11-14 und einen pharmazeutisch akzeptablen Träger oder ein Verdünnungsmittel enthält.

16. Salz nach einem der Ansprüche 1-10, freie Base nach einem der Ansprüche 11-14 oder pharmazeutische Zusammensetzung nach Anspruch 15 zur Verwendung in einem Verfahren zur Behandlung von Krebs bei einem Individuum, wobei optional:
(i) der genannte Krebs nichtkleinzelliger Lungenkrebs ist;
(ii) das genannte Individuum mindestens eine Mutation in dem EGFR-Gen aufweist, die zur Expression eines EGFR-Enzyms mit Aminosäuremodifikation führt, ausgewählt aus der Gruppe, die aus L858R, T790M, C797S und Kombinationen davon besteht, wobei das genannte EGFR-Gen optional Aminosäuremodifikationen von L858R, T790M und C797S aufweist; und/oder
(iii) das Verfahren ferner das Verabreichen einer wirksamen Menge von Afatinib, Osimertinib, Erlotinib oder Gefitinib an das genannte Individuum umfasst.

17. Salz nach einem der Ansprüche 1-10, freie Base nach einem der Ansprüche 11-14 oder pharmazeutische Zusammensetzung nach Anspruch 15 zur Verwendung in einem Verfahren zur Hemmung von epidermalem Wachstumsfaktorrezeptor (EGFR) in einem Individuum, das dies benötigt.

## Revendications

1. Sel du Composé (I), représenté par la formule structurale suivante : 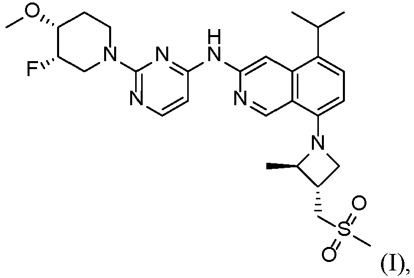 dans laquelle le sel est un sel succinate et le rapport molaire entre le Composé (I) et l'acide succinique est de 1:0,5, ou dans laquelle le sel est un sel glutarate et le rapport molaire entre le Composé (I) et l'acide glutarique est de 1:0,5, ou dans laquelle le sel est un sel fumarate et le rapport molaire entre le Composé (I) et l'acide fumarique est de 1:1.

2. Sel succinate selon la revendication 1, le sel succinate étant cristallin.

3. Sel succinate selon la revendication 2, ledit sel succinate cristallin étant une forme cristalline C, **caractérisée par** :
(i) un diagramme de diffraction sur poudre des rayons X qui comprend au moins trois ou quatre pics choisis parmi 4,5 °, 8,9 °, 9,3 °, 15,3 ° et 17,8 ° ± 0,2 en 2θ ;
(ii) un diagramme de diffraction sur poudre des rayons X qui comprend des pics à 4,5 °, 8,9 °, 9,3 °, 15,3 ° et 17,8 ° ± 0,2 en 2θ ;
(iii) un diagramme de diffraction sur poudre des rayons X qui comprend au moins trois, quatre, cinq, six, sept, huit, neuf ou dix pics choisis parmi 4,5 °, 8,9 °, 9,3 °, 13,0 °, 15,3 °, 16,8 °, 17,8 °, 18,1 °, 18,5 ° et 22,3 ° ± 0,2 en 2θ ;
(iv) un diagramme de diffraction sur poudre des rayons X qui comprend des pics à 4,5 ° 6,7 °, 8,9 °, 9,3 °, 11,1 °, 12,3 °, 13,0 °, 14,4 °, 15,3 °, 16,3 °, 16,8 °, 17,8 °, 18,1 °, 18,5 °, 20,5 °, 22,3 ° et 26,0 ° ± 0,2 en 2θ ; ou
(v) un diagramme de diffraction sur poudre des rayons X sensiblement similaire à la Figure 1 ; et/ou
ledit sel succinate cristallin étant la Forme C, **caractérisée par** une calorimétrie différentielle à balayage comportant une température de début de 175 ± 2 °C ; et/ou
ledit sel succinate cristallin étant la Forme C, **caractérisée par** une analyse thermogravimétrique (ATG) sensiblement similaire à la Figure 2 ; et/ou
au moins 90 % en poids du sel succinate étant la forme cristalline C.

4. Sel succinate selon l'une quelconque des revendications 1 à 3, obtenu par un procédé comprenant :
la combinaison du Composé (I) et d'acide succinique dans de l'acétate d'éthyle ;
la collecte dudit sel succinate du Composé (I) ;
l'ajout de 2-propanol audit sel succinate du Composé (I) ; et
une répétition de la collecte dudit sel succinate du Composé (I).

5. Sel glutarate selon la revendication 1, ledit sel glutarate étant cristallin.

6. Sel glutarate selon la revendication 5, ledit sel glutarate cristallin étant la Forme D, **caractérisée par** :
i) un diagramme de diffraction sur poudre des rayons X qui comprend au moins trois ou quatre pics choisis parmi 8,8 °, 14,8 °, 16,1 °, 18,3 ° et 18,7 ° ± 0,2 en 2θ ;
(ii) un diagramme de diffraction sur poudre des rayons X qui comprend des pics à 8,8 °, 14,8 °, 16,1 °, 18,3 ° et 18,7 ° ± 0,2 en 2θ ;
(iii) un diagramme de diffraction sur poudre des rayons X qui comprend au moins trois, quatre, cinq, six ou sept pics choisis parmi 7,4 °, 8,8 °, 12,3 °, 14,8 °, 16,1 °, 18,3 ° et 18,7 ° ± 0,2 en 2θ ;
(iv) un diagramme de diffraction sur poudre des rayons X qui comprend des pics à 6,6 °, 7,4 °, 8,8 °, 12,3 °, 12,9 °, 14,8 °, 16,1 °, 18,3 °, 18,7 °, 20,0 ° et 22,2 ° ± 0,2 en 2θ ; ou
(v) un diagramme de diffraction sur poudre des rayons X sensiblement identique à la Figure 4 ; et/ou
ledit sel glutarate cristallin étant la Forme D, **caractérisée par** une calorimétrie différentielle à balayage comportant une température de début de 142 ± 2 °C ; et/ou
ledit sel glutarate cristallin étant la Forme D, **caractérisée par** une analyse thermogravimétrique (ATG) sensiblement similaire à la Figure 5 ; et/ou
au moins 90 % en poids du sel glutarate étant la forme cristalline D.

7. Sel glutarate selon l'une quelconque des revendications 1, 5 et 6, obtenu par un procédé comprenant :
la combinaison du Composé (I) et d'acide glutarique dans de l'acétate d'éthyle ;
la collecte dudit sel glutarate du Composé (I).

8. Sel fumarate selon la revendication 1, ledit sel fumarate étant cristallin.

9. Sel fumarate selon la revendication 8, ledit sel fumarate cristallin étant la Forme E, **caractérisée par** :
(i) un diagramme de diffraction sur poudre des rayons X qui comprend au moins trois, quatre ou cinq pics choisis parmi 6,3 °, 8,5 °, 9,0 °, 14,5 °, 15,7 ° et 18,0 ° ± 0,2 en 2θ ;
(ii) un diagramme de diffraction sur poudre des rayons X qui comprend des pics à 6,3 °, 8,5 °, 9,0 °, 14,5 °, 15,7 ° et 18,0 ° ± 0,2 en 2θ ;
(iii) un diagramme de diffraction sur poudre des rayons X qui comprend au moins trois, quatre, cinq, six, sept, huit, neuf ou dix pics choisis parmi 6,3 °, 8,5 °, 9,0 °, 12,1 °, 14,5 °, 15,7 °, 18,0 °, 19,7 °, 20,1 ° et 21,9 ° ± 0,2 en 2θ ;
(iv) un diagramme de diffraction sur poudre des rayons X qui comprend des pics à 6,3 °, 8,5 °, 9,0 °, 12,1 °, 14,5 °, 15,1 °, 15,2 °, 15,4 °, 15,7 °, 18,0 °, 18,2 °, 18,9 °, 19,3 °, 19,7 °, 20,1 °, 20,6 °, 20,7 °, 21,3 ° et 21,9° ± 0,2 en 2θ ; ou
(v) un diagramme de diffraction sur poudre des rayons X sensiblement similaire à la Figure 7 ; et/ou
ledit sel fumarate cristallin étant la Forme E, **caractérisée par** une calorimétrie différentielle à balayage comportant une température de début de 164 ± 3 °C ; et/ou
ledit sel fumarate cristallin étant la Forme E, **caractérisée par** une analyse thermogravimétrique (ATG) sensiblement similaire à la Figure 8 ; et/ou
au moins 90 % en poids du sel fumarate étant la forme cristalline E.

10. Sel fumarate selon l'une quelconque des revendications 1, 8 et 9, obtenu par un procédé comprenant :
la combinaison du Composé (I) et d'acide fumarique dans de l'acétate d'éthyle ;
l'élimination dudit acétate d'éthyle pour produire ledit sel fumarate du Composé (I) ;
l'ajout de trifluoroéthanol audit sel fumarate du Composé (I) ;
l'élimination dudit trifluoroéthanol pour produire ledit sel fumarate du Composé (I) ; et
l'ajout d'acétate d'éthyle audit sel fumarate du Composé (I).

11. Forme cristalline B de base libre du Composé (I), représentée par la formule structurale suivante : 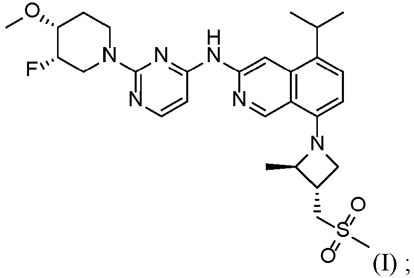
ladite Forme B étant **caractérisée par** un diagramme de diffraction sur poudre des rayons X qui comprend au moins trois, quatre ou cinq pics choisis parmi 5,1 °, 12,2 °, 13,5 °, 16,6 ° et 20,1 ° ± 0,2 en 2θ, ladite Forme B étant optionnellement **caractérisée en outre par** :
(i) un diagramme de diffraction sur poudre des rayons X qui comprend au moins trois, quatre, cinq, six, sept, huit, neuf, dix ou onze pics choisis parmi 5,1 °, 12,2 °, 13,5 °, 16,3 °, 16,6 °, 19,5 °, 20,1 °, 20,4 °, 21,4 °, 22,7 ° et 25,2 ° ± 0,2 en 2θ ;
(ii) un diagramme de diffraction sur poudre des rayons X qui comprend des pics à 5,1 °, 12,2 °, 13,5 °, 15,2 °, 16,3 °, 16,6 °, 17,9 °, 19,5 °, 20,1 °, 20,4 °, 20,7 °, 20,9 °, 21,4 °, 22,7 °, 25,2 ° et 26,3 ° ± 0,2 en 2θ ; ou
(iii) un diagramme de diffraction sur poudre des rayons X sensiblement similaire à la Figure 12 ; et/ou
ladite Forme B étant optionnellement **caractérisée en outre par** une calorimétrie différentielle à balayage comportant une température de début de 158 ± 2 °C ; et/ou
ladite Forme B étant optionnellement **caractérisée en outre par** une analyse thermogravimétrique (ATG) sensiblement similaire à la Figure 13 ; et/ou optionnellement, au moins 90 % en poids de la forme cristalline étant la forme cristalline B.

12. Forme cristalline B de base libre selon la revendication 11, obtenue par un procédé comprenant :
l'ajout du Composé (I) à du diméthylacétamide pour former un mélange ; et
l'ajout dudit mélange à de l'eau pour obtenir la forme cristalline B de base libre du Composé (I).

13. Forme amorphe de base libre du Composé (I) représentée par la formule structurale suivante : 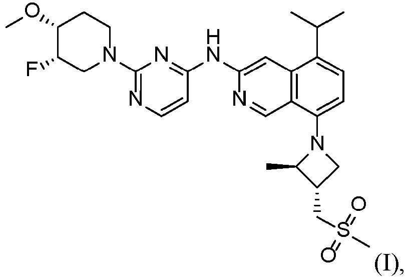 ladite forme amorphe étant **caractérisée par** une calorimétrie différentielle à balayage comportant une température de début de 157 ± 2 °C.

14. Forme amorphe selon la revendication 13, obtenue par un procédé comprenant :
l'ajout d'un mélange d'acétonitrile et d'eau au Composé (I) pour former une solution ;
une filtration de ladite solution ;
une congélation de ladite solution ; et
une lyophilisation de ladite solution congelée pour obtenir ledit Composé amorphe (I).

15. Composition pharmaceutique comprenant ledit sel selon l'une quelconque des revendications 1 à 10, ou ladite base libre selon l'une quelconque des revendications 11 à 14, et un support ou un diluant pharmaceutiquement acceptable.

16. Sel selon l'une quelconque des revendications 1 à 10, base libre selon l'une quelconque des revendications 11 à 14, ou composition pharmaceutique selon la revendication 15, destiné(e) à une utilisation dans une méthode de traitement d'un cancer chez un sujet, optionnellement :
(i) ledit cancer étant un cancer du poumon non à petites cellules ;
(ii) ledit sujet présentant au moins une mutation dans le gène EGFR qui entraîne l'expression d'une enzyme EGFR comportant une modification d'acide aminé sélectionnée dans le groupe constitué de L858R, T790M, C797S, et de combinaisons de celles-ci, ledit gène EGFR présentant optionnellement des modifications d'acides aminés L858R, T790M et C797S ; et/ou
(iii) le procédé comprenant en outre l'administration audit sujet d'une quantité efficace d'afatinib, d'osimertinib, d'erlotinib ou de gefitinib.

17. Sel selon l'une quelconque des revendications 1 à 10, base libre selon l'une quelconque des revendications 11 à 14, ou composition pharmaceutique selon la revendication 15, destiné(e) à une utilisation dans une méthode d'inhibition du récepteur du facteur de croissance épidermique (EGFR) chez un sujet qui en a besoin.
